(19) Europäisches Patentamt
European Patent Office
Office européen des brevets

(11) **EP 4 725 912 A1**

(12) **EUROPEAN PATENT APPLICATION**

(43) Date of publication:
**15.04.2026  Bulletin 2026/16**

(21) Application number: **24206010.1**

(22) Date of filing: **11.10.2024**

(51) International Patent Classification (IPC):
**C02F 1/00** $^{(2023.01)}$    **C02F 1/42** $^{(2023.01)}$
**G01N 33/18** $^{(2006.01)}$    **C02F 1/28** $^{(2023.01)}$

(52) Cooperative Patent Classification (CPC):
**C02F 1/008; C02F 1/42; G01N 33/1853;**
C02F 1/283; C02F 2001/425; C02F 2209/008;
C02F 2209/02; C02F 2209/05; C02F 2209/06;
C02F 2209/40; C02F 2209/445; C02F 2301/043;
C02F 2303/14

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **BRITA SE**
**65232 Taunusstein (DE)**

(72) Inventors:
• **WEIDNER, Peter**
  **92444 Rötz (DE)**
• **Meyer, Fabian**
  **65618 Niederselters (DE)**

(54) **METHOD AND SYSTEMS FOR DETERMINATION OF TREATMENT CAPACITIES OF A SET OF LIQUID TREATMENT SYSTEMS**

(57)     In a set of liquid treatment systems (10a-c,15a-c,20) connected in parallel to a common source (6,7,8,9) of liquid to be treated, each liquid treatment system (10a-c,15a-c,20) comprises a liquid treatment device (12a-c,17a-c,22) and an operational data processing system comprising at least an interface to an associated sensor (38) for measuring at least one parameter for, at least in combination with setting information that is information on at least one setting ($x$;82) of the liquid treatment system (10a-c,15a-c,20), determining a value of a concentration measure ($H$) that is a measure of a concentration of at least one component changeable by the liquid treatment device (12a-c,17a-c,22) in liquid flowing through the liquid treatment device (12a-c,17a-c,22). The operational data processing system is configured to carry out a process (43,45,46;58,60,61;81,83,86,87) associated with operating the liquid treatment system (10a-c,15a-c,20). A method of processing data obtained from the set of liquid treatment systems (10a-c,15a-c,20) comprises: obtaining from multiple ones of the liquid treatment systems (10a-c,15a-c,20) in the set local data (49;64;73). The local data is selected from the group consisting of: (i) a value of the concentration measure ($H$) determined by the operational data processing system of that liquid treatment system (10a-c,15a-c,20) on the basis of at least one value of the parameter obtained from the sensor (38) associated with the operational data processing system; and (ii) at least one value of the parameter determined by the sensor (38) associated with the operational data processing system of that liquid treatment system (10a-c,15a-c,20) and any setting information required to determine a value of the concentration measure ($H$) on the basis of the at least one value of the parameter. The local data (49;64;73) obtained from the multiple liquid treatment systems (10a-c,15a-c,20) is processed to determine a consensus value (54;59;79) of the concentration measure ($H$). At least one of the consensus value and a parameter value derived from the consensus value is provided as input to the process (43,45,46;58,60,61;81,83,86,87) performed by the operational data processing system of at least one of the liquid treatment systems (10a-c,15a-c,20) in the set.

Fig. 4

**Description**

The invention relates to a method of processing data obtained from a set of liquid treatment systems connected in parallel to a common source of liquid to be treated.

The invention also relates to a method controlling operation of a liquid treatment system comprised in a set of liquid treatment systems connected in parallel to a common source of liquid to be treated.

The invention also relates to a system for processing data obtained from a set of liquid treatment systems connected in parallel to a common source of liquid to be treated.

The invention also relates to a system for processing operational data of a liquid treatment system.

The invention also relates to computer programs.

WO 2013/166069 A1 discloses a water filtration device comprising a communications module, a controller, a replaceable filter and a flow rate modifier. A database is located in the cloud and comprises information regarding water contaminant profiles in at least one municipality. The database data may come from the municipality, from independent water analysis or from a contaminant analysis module in the water filtration device itself. In an embodiment, a smart phone has an application installed that can communicate with the database and with the water filtration device. The smart phone contacts the database to determine the water contaminant profile at the user's location and transmits the water contaminant profile to the water filtration device. The water filtration device then adjusts its operation in response to the water contaminant profile. For example, it may adjust the filter life expectancy dependent on the water contaminant levels. It may change the flow rate through the filter.

A problem associated with using contaminant profiles that are location-based is that premises located relatively close together, e.g. on opposite sides of the same street, may be supplied with mains water from different sources. A problem associated with using a contaminant profile determined by the water filtration device itself occurs when multiple such filtration devices are in use at the same premises. Due to variations within a specified tolerance band for the determination, adjustments of the water filtration device settings or determinations of the filter life expectancy will differ between the filtration devices at the same premises. The result is that a need to replace the filter will be signalled at different times. Since replacement often involves calling out a service technician, this is inefficient.

It is an object of the invention to provide methods, systems and computer programs of the types defined above in the opening paragraphs that allow for efficient maintenance of a set of liquid treatment systems installed at a particular site.

The problem is solved according to a first aspect of the invention by providing a method of processing data obtained from a set of liquid treatment systems connected in parallel to a common source of liquid to be treated,

wherein each liquid treatment system comprises:

a liquid treatment device; and
an operational data processing system comprising:

at least an interface to an associated sensor for measuring at least one parameter for, at least in combination with setting information that is information on at least one setting of the liquid treatment system, determining a value of a concentration measure that is a measure of a concentration of at least one component changeable by the liquid treatment device in liquid flowing through the liquid treatment device,
wherein the operational data processing system is configured to carry out a process associated with operating the liquid treatment system,

wherein the method comprises:

obtaining from multiple ones of the liquid treatment systems in the set local data, wherein the local data is selected from the group consisting of:

(i) a local value of the concentration measure ($H$) determined by the operational data processing system of that liquid treatment system on the basis of at least one value of the parameter determined by the sensor associated with the operational data processing system; and
(ii) at least one value of the parameter obtained from the sensor associated with the operational data processing system of that liquid treatment system and any setting information required to determine a value of the concentration measure on the basis of the at least one value of the parameter;

processing the local data obtained from the multiple liquid treatment systems to determine a consensus value of the concentration measure; and
providing at least one of the consensus value and a parameter value derived from the consensus value as input to the process performed by the operational data processing system of at least one of the liquid treatment systems in the set.

The method can be carried out by a data processing system other than a data processing system associated with or comprised in a specific one of the liquid treatment systems or conversely by a data processing system comprised in or associated with a specific one of the liquid processing systems, e.g. a data processing system also forming an operational data processing system of one of the liquid treatment systems.

The liquid treatment systems may each be of the type comprising a head device, the head device comprising a connector for connecting the head device to a conduit in liquid communication with the source of liquid and a mechanical interface for co-operating with a mechanical interface of a replaceable liquid treatment cartridge to couple the liquid treatment cartridge to the head device, the head device being configured to divert through the replaceable liquid treatment cartridge at least a fraction of liquid flowing into the head device through the connector. In such embodiments, the operational data processing system may be comprised in the head device. The sensor may additionally be comprised in the head device or the head device may comprise the interface to the sensor. The system carrying out the method may be one of the head devices or a general-purpose computer in communication with the head devices.

The set of liquid treatment systems is connected in parallel to a common source of liquid to be treated, e.g. a common mains water connection. Thus, it is to be expected that the local value of the concentration measure determined by each liquid treatment system of the set is the same. In practice this will not be the case. By processing the local data obtained from the multiple liquid treatment systems in the set to determine a consensus value of the concentration measure, a relatively accurate consensus value is obtained. By providing at least one of the consensus value and a parameter value derived from the consensus value as input to the process performed by the operational data processing system of at least one of the liquid treatment systems in the set, the processes are carried out on the basis of the same data. Whether the process is an actual process of controlling operation of the liquid treatment systems or a monitoring process for determining a need for maintenance or both, an improvement in synchronisation of maintenance schedules for the liquid treatment systems at a common site can be achieved.

Each liquid treatment system in the set comprises a liquid treatment device for the treatment of liquid, e.g. by means of a physical, physico-chemical, biological or chemical process. The treatment is apt to change a concentration of at least one component in liquid flowing through the liquid treatment device.

The concentration may increase or decrease. Components may be substances, e.g. compounds or ions. Components may be biological contaminants. Each liquid treatment system comprises a sensor for measuring at least one parameter for, at least in combination with setting information that is information on at least one setting of the liquid treatment system, determining a value of a concentration measure that is a measure of a concentration of the at least one component changeable by the liquid treatment device in liquid flowing through the liquid treatment device. In the case of an aqueous liquid, the parameter may be electrical conductivity, electrical conductivity adjusted for deviations from a standard value of temperature of the liquid, optionally approximated by ambient temperature, pH, ion concentration as measured by an ion-selective sensor, etc.

The operational data processing system is a data processing system for processing operational data of the liquid treatment system in which the operational data processing system is comprised. Such data is data informing on or suitable for use in controlling the operation of the liquid treatment system or both. Thus, the process associated with operating the liquid treatment system may be a process providing information on a state of operation of the liquid treatment system as output. Alternatively, the process may be a process of adjusting a setting of the liquid treatment system.

The method comprises obtaining local data from multiple ones of the liquid treatment systems in the set. Local data is data specific to one of the liquid treatment systems. Thus, this step comprises obtaining respective sets of local data from each of the multiple liquid treatment systems. Where the method is carried out by a data processing system comprised in or associated with one of the liquid treatment systems, the local data may be local data obtained in or from that liquid treatment system and local data obtained from at least one other of the liquid treatment systems in the set.

Each obtained set of local data comprises at least one of: (i) a local value of the concentration measure determined by the operational data processing system of that liquid treatment system on the basis of at least one value of the parameter determined by the sensor of the operational data processing system; and (ii)at least one value of the parameter determined by the sensor of the operational data processing system of that liquid treatment system and any setting information required to determine a value of the concentration measure on the basis of the at least one value of the parameter. Thus, each set of local data comprises either the local value or enough data to allow a local value to be calculated. In the latter alternative, the local data in other words comprises at least one value of the parameter and, if necessary to calculate the value of the concentration measure, the setting information needed to calculate the value of the concentration measure from the at least one parameter value. Setting information may be a value corresponding exactly to a setting of a device or a value quantifying the effect of the setting on the liquid as processed by the liquid treatment system. For example, the setting information may be a target temperature setting of a heater or the measured temperature of the liquid, the parameter value being, for example, a value of the electrical conductivity of the liquid. Another example would be where the treated liquid is blended with untreated liquid and the parameter value pertains to the blend. The setting information may then comprise a value quantifying the fraction of treated or the fraction of untreated liquid in the blend or the setting information may be a value quantifying a setting of the device used to set the ratio of blended to unblended liquid.

The method comprises processing the local data obtained from the multiple liquid treatment systems to determine a consensus value of the concentration measure. All the data is processed, but the consensus value need not be the result of a calculation in which the local data from each of the multiple liquid treatment systems is an input. Examples would be where the consensus value is a median value or where local data from certain liquid treatment systems is evaluated and then discarded, e.g. in a process of outlier removal. Another example would be where the process is carried out by a trained Artificial Neural Network.

The method also comprises providing at least one of the consensus value and a parameter value derived from the consensus value as input to the process performed by the operational data processing system of at least one of the liquid treatment systems in the set. The parameter value derived from the consensus value may, for example, be a device setting or an instruction to a display device or the like.

In an embodiment of the method, the step of obtaining local data from multiple ones of the liquid treatment systems comprises receiving the local data via at least one Wide Area Network, and the step of providing at least one of the consensus value and a parameter value derived from the consensus value as input to the process performed by the operational data processing system of at least one of the liquid treatment systems in the set, comprises communicating the value(s) to the at least one liquid treatment system via at least one Wide Area Network.

In this embodiment, the method of processing data obtained from the set of liquid treatment systems involves a remote data processing system. This remote data processing system can carry out at least the step of processing the local data obtained from the multiple liquid treatment systems to determine a consensus value of the concentration measure. An effect is that the operational data processing systems of at least some of the liquid treatment systems can be relatively simple. It is noted that they need not themselves be directly connected to the Wide Area Network, but may be connected thereto through a gateway device. The Wide Area Network may be based on the Internet Protocol at the network layer (and e.g. Transmission Control Protocol at the transport layer). The Wide Area Network may comprise wired and/or cellular wireless networks.

In another embodiment, the step of obtaining local data from multiple ones of the liquid treatment systems comprises the operational data system of one of the liquid treatment systems receiving local data originating from the operational data system of at least one other of the liquid treatment systems via at least one data communication network.

In this embodiment, the set of liquid treatment systems operates relatively autonomously.

In a particular example of this embodiment, the at least one data communication network comprises a Wide Area Network.

In this example, there is no need to set up a local data communication network for the liquid treatment systems in the set to exchange data. They can each be equipped with a wireless transceiver for connecting to a cellular communications network, for example. Alternatively, they can each be provided with a wired or short-range wireless connection to a device connected to the Wide Area Network.

In a particular example of any embodiment in which the step of obtaining local data from multiple ones of the liquid treatment systems comprises the operational data system of one of the liquid treatment systems receiving local data originating from the operational data system of at least one other of the liquid treatment systems via at least one data communication network and the at least one data communication network comprises a Wide Area Network, the local data originating from the at least one other of the liquid treatment systems is received from a remote server system configured to collate local data communicated to the remote server system by the liquid treatment systems.

In this example, the liquid treatment systems need only be configured to communicate local data to the remote server system. The remote server system can, for example, be a system operated by a supplier or operator of a much larger number of liquid treatment systems than those comprised in the set. The remote server system may be configured to identify the members of the set and thus determine how the local data are to be collated and to which liquid treatment systems the collated data is to be distributed.

In an example of any embodiment of the method in which the step of obtaining local data from multiple ones of the liquid treatment systems comprises the operational data system of one of the liquid treatment systems receiving local data originating from the operational data system of at least one other of the liquid treatment systems via at least one data communication network, the at least one data communication network comprises at least one of a Local Area Network and a Personal Area Network, and the local data are received via respective links through the at least one data communication network, established between the at least one other liquid treatment system and the liquid treatment system of which the operational data processing system receives the local data.

This embodiment minimises data transfers and requires few additional computational resources next to those of the operational data processing systems of the liquid treatment systems.

An embodiment of the method comprises identifying the members of the set from among a larger group of liquid treatment systems.

The larger group can in particular comprise liquid treatment systems connected to a different source of liquid to be treated or connected to the common source via a device liable to affect the concentration of the at least one component of which the concentration is changeable by the liquid treatment device in liquid flowing through the liquid treatment device. Those are excluded from the identified set.

An example of any embodiment that comprises identifying the members of the set from among a larger group of liquid treatment systems comprises determining respective locations of liquid treatment systems from which local data are obtained and identifying the members of the set based on proximity.

This may comprise one of the liquid treatment systems determining which other liquid treatment systems are within range of a wireless transceiver of that liquid treatment system, for example. Alternatively, the liquid treatment systems may be equipped with a positioning device and configured to communicate a location determined by the positioning device to the entity carrying out the step of identifying the members of the set.

Another example of any embodiment that comprises identifying the members of the set from among a larger group of liquid treatment systems comprises determining respective values of tagging data associated with liquid treatment systems from which local data are obtained and identifying the members of the set based on correspondence of tagging data.

The tagging data may be programmed into the liquid treatment systems when they are installed, e.g. programmed by a service technician or user.

In a particular example of any embodiment that comprises identifying the members of the set from among a larger group of liquid treatment systems and that comprises determining respective values of tagging data associated with liquid treatment systems from which local data are obtained and identifying the members of the set based on correspondence of tagging data, determining a value of tagging data comprises querying a database, wherein the database is accessible to a user, e.g. remotely accessible through a Wide Area Network, to enable the user to associate tagging data with liquid treatment systems.

In this example, the liquid treatment systems need not themselves be provided with the tagging data or communicate this tagging data. They need merely be identifiable, such that the tagging data associated with them can be retrieved from the database.

In another example of any method that comprises identifying the members of the set from among a larger group of liquid treatment systems, identifying the members of the set comprises at least one of:

detecting their presence as nodes in a communication network and
identifying them as members on the basis of their address in the communication network.

In the first case, for example, all liquid treatment systems that are nodes in a Wireless Local Area Network may be identified as members of the set. In the second case, all those allocated a network address within a particular range or all those communicating via a gateway device with a particular network address may be identified. It is also possible to identify liquid treatment systems by means of a network address at the link layer.

According to a second aspect of the invention, there is provided a method of controlling operation of a liquid treatment system comprised in a set of liquid treatment systems connected in parallel to a common source of liquid to be treated,

wherein each liquid treatment system comprises:

a liquid treatment device; and
an operational data processing system comprising:
at least an interface to an associated sensor for measuring at least one parameter for, at least in combination with setting information that is information on at least one setting of the liquid treatment system, determining a value of a concentration measure that is a measure of a concentration of at least one component changeable by the liquid treatment device in liquid flowing through the liquid treatment device,

wherein the method comprises:

the operational data processing system of one of the liquid treatment systems obtaining at least one value of the parameter from the sensor and any setting information required to determine a value of the concentration measure on the basis of the at least one value of the parameter,
the operational data processing system providing local data as input to a data processing system, wherein the local data is selected from the group consisting of:

(i) a local value of the concentration measure determined on the basis of at least one value of the parameter obtained from the sensor associated with the operational data processing system; and

(ii) the at least one value of the parameter obtained from the sensor and any setting information required to determine a value of the concentration measure on the basis of the at least one value of the parameter,

wherein the data processing system to which the local data is provided as input is configured to obtain respective local data from multiple ones of the liquid treatment systems in the set and to use the obtained local data to determine a consensus value of the concentration measure;

the operational data processing system obtaining at least one of the consensus value and a parameter value derived from that consensus value; and

the operational data processing system using the obtained value as input in a process associated with operating the liquid treatment system.

The data processing system configured to obtain respective local data from multiple ones of the liquid treatment systems in the set and to use the obtained local data to determine a consensus value of the concentration measure may be configured to carry out a method according to the invention of facilitating operation of a set of liquid treatment systems.

The set of liquid treatment systems is connected in parallel to a common source of liquid to be treated, e.g. a common mains water connection. Thus, it is to be expected that the local value of the concentration measure determined by each liquid treatment system of the set is the same. In practice this will not be the case. By obtaining at least one of the consensus value and a parameter value derived from that consensus value and using the obtained value as input in a process associated with operating the liquid treatment system, each liquid treatment system in the set that is operated in accordance with the method is operated on the basis of the same data. Whether the process is an actual process of controlling operation of the liquid treatment systems or a monitoring process for determining a need for maintenance or both, an improvement in synchronisation of maintenance schedules for the liquid treatment systems at a common site can be achieved.

The liquid treatment systems may each be of the type comprising a head device, the head device comprising a connector for connecting the head device to a conduit in liquid communication with the source of liquid and a mechanical interface for co-operating with a mechanical interface of a replaceable liquid treatment cartridge to couple the liquid treatment cartridge to the head device, the head device being configured to divert through the replaceable liquid treatment cartridge at least a fraction of liquid flowing into the head device through the connector. In such embodiments, the operational data processing system may be comprised in the head device. The sensor may additionally be comprised in the head device or the head device may comprise the interface to the sensor. The system carrying out the steps of the method may be one of the head devices.

Each liquid treatment in the set comprises a liquid treatment device for the treatment of liquid, e.g. by means of a physical, physico-chemical, biological or chemical process. The treatment is apt to change a concentration of at least one component in liquid flowing through the liquid treatment device. The concentration may increase or decrease. Components may be substances, e.g. compounds or ions. Components may be biological contaminants. Each liquid treatment system comprises a sensor for measuring at least one parameter for, at least in combination with setting information that is information on at least one setting of the liquid treatment system, determining a value of a concentration measure that is a measure of a concentration of the at least one component changeable by the liquid treatment device in liquid flowing through the liquid treatment device. In the case of an aqueous liquid, the parameter may be electrical conductivity, electrical conductivity adjusted for deviations from a standard value of temperature of the liquid, optionally approximated by ambient temperature, pH, ion concentration as measured by an ion-selective sensor, etc.

The process associated with operating the liquid treatment system may be a process providing as output information on a state of operation of the liquid treatment system or a process of adjusting a setting of the liquid treatment system.

Providing local data as input to the data processing system may comprise communicating the local data to that data processing system. The system carrying out the method and the data processing system arranged to process local data may be the same system for one of the liquid treatment systems in the set. In that case, providing as input means no more than actually calculating the local data.

Each set of local data comprises at least one of: (i) a local value of the concentration measure determined by the operational data processing system of that liquid treatment system on the basis of at least one value of the parameter determined by the sensor of the operational data processing system; and (ii) at least one value of the parameter determined by the sensor of the operational data processing system of that liquid treatment system and any setting information required to determine a value of the concentration measure on the basis of the at least one value of the parameter. Thus, each set of local data comprises either the local value or all data necessary to calculate one local value. Setting information may be a value corresponding exactly to a setting of a device or a value quantifying the effect of the setting on the liquid as processed by the liquid treatment system. For example, the setting information may be a target temperature setting of a heater or the measured temperature of the liquid, the parameter value being, for example, a value of the electrical conductivity of the liquid. Another example would be where the treated liquid is blended with untreated liquid and the parameter value pertains to the blend. The setting information may then comprise a value quantifying the fraction of treated or the fraction of untreated liquid in the blend or the setting information may be a value quantifying a setting of the device used to set the ratio of blended to unblended liquid.

The data processing system that is configured to obtain respective local data from multiple ones of the liquid treatment systems in the set and to use the obtained local data to determine a consensus value of the concentration measure is configured to process all the data. It is not necessarily the case that the consensus value is the result of a calculation in which the local data from each of the multiple liquid treatment systems is an input. Examples would be where the consensus value is a median value or where local data from certain liquid treatment systems is evaluated and then discarded, e.g. in a process of outlier removal. Another example would be where the process is carried out by a trained Artificial Neural Network.

In an embodiment of the method, the step of providing local data as input to a data processing system comprises communicating the local data to a remote server system via at least one Wide Area Network.

In this embodiment, a user or service technician need not set up the liquid treatment systems in the set to communicate directly amongst themselves. They can be configured by a supplier to connect to a remote server system operated by the supplier or on behalf of the supplier, for example.

In an embodiment of the method, the step of obtaining at least one of the consensus value and a parameter value derived from the consensus value comprises receiving the value via at least one communication network, e.g. the at least one Wide Area Network.

The system carrying out the method need not therefore itself calculate the consensus value or the parameter value derived from the consensus value.

In an example of any embodiment of the method in which the step of providing local data as input to a data processing system comprises communicating the local data to a remote server system via at least one Wide Area Network and the step of obtaining at least one of the consensus value and a parameter value derived from the consensus value comprises receiving the value via at least one communication network, e.g. the at least one Wide Area Network, the remote server system comprises the data processing system configured to obtain respective local data from multiple ones of the liquid treatment systems in the set and to use the obtained local data to determine the consensus value of the concentration measure.

Thus, the liquid treatment systems need only communicate the local data to the remote server system and they receive the consensus value or the parameter value derived from the consensus value back.

In another embodiment of the method of operating a liquid treatment system, the step of providing local data as input to a data processing system comprises communicating the local data to the operational data processing system of at least one of the liquid treatment systems, which operational data processing system comprises the data processing system configured to obtain respective local data from multiple ones of the liquid treatment systems in the set and to use the obtained local data to determine the consensus value of the concentration measure.

Thus, only the remote server system need comprise the computing resources necessary to calculate the consensus value or the parameter value derived from the consensus value.

In an example of any embodiment in which the step of providing local data as input to a data processing system comprises communicating the local data to a remote server system via at least one Wide Area Network and the step of providing local data as input to a data processing system comprises communicating the local data to the operational data processing system of at least one of the liquid treatment systems, which operational data processing system comprises the data processing system configured to obtain respective local data from multiple ones of the liquid treatment systems in the set and to use the obtained local data to determine the consensus value of the concentration measure, the remote server system is configured to collate local data received from the multiple liquid treatment systems and to communicate the collated local data to the operational data processing system comprising the data processing system configured to obtain respective local data from multiple ones of the liquid treatment systems in the set and to use the obtained local data to determine the consensus value of the concentration measure.

In an embodiment of the method of operating the liquid treatment system, obtaining at least one of the consensus value and a parameter value derived from that consensus value comprises obtaining respective local data from multiple ones of the liquid treatment systems in the set and using the obtained local data to determine the consensus value of the concentration measure.

In this embodiment, the system carrying out the method also calculates at least one of the consensus value and a parameter value derived from that consensus value.

In an example of this embodiment, obtaining the respective local data from multiple ones of the liquid treatment systems in the set comprises the operational data processing system of one of the liquid treatment systems receiving local data originating from the operational data processing system of at least one other of the liquid treatment systems via at least one data communication network.

In an example of any embodiment of the method in which obtaining at least one of the consensus value and a parameter value derived from that consensus value comprises obtaining respective local data from multiple ones of the liquid treatment systems in the set and using the obtained local data to determine the consensus value of the concentration measure and obtaining the respective local data from multiple ones of the liquid treatment systems in the set comprises the operational data processing system of one of the liquid treatment systems receiving local data originating from the operational data processing system of at least one other of the liquid treatment systems via at least one data communication network, the at least one data communication network comprises at least one Wide Area Network, and receiving the local data originating from the operational data processing system of at least one other of the multiple liquid treatment systems via at least one data communication network comprises receiving the local data from a remote server system configured to collate local data received from the liquid treatment systems.

In another example of any embodiment of the method in which obtaining at least one of the consensus value and a parameter value derived from that consensus value comprises obtaining respective local data from multiple ones of the liquid treatment systems in the set and using the obtained local data to determine the consensus value of the concentration measure and obtaining the respective local data from multiple ones of the liquid treatment systems in the set comprises the operational data processing system of one of the liquid treatment systems receiving local data originating from the operational data processing system of at least one other of the liquid treatment systems via at least one data communication network, the at least one data communication network comprises at least one of a Local Area Network and a Personal Area Network, and the local data is received via respective links through the at least one data communication network established between the at least one other liquid treatment system and the liquid treatment system of which the operational data processing system receives the local data.

In an embodiment of the method of controlling operation of a liquid treatment system, the step of using the obtained value as input in the process associated with operating the liquid treatment system is carried out on condition that the obtained value deviates by less than a certain amount from the local value or the parameter as derived from the local value, respectively.

The certain amount may be an absolute or relative amount. This embodiment is able to deal with the eventuality that the members of the set have been incorrectly identified. This can in particular occur where a user or service technician allocated tagging data to the liquid treatment system and this tagging data is used to identify the members of the set.

A similar effect is obtained in an embodiment of the method that further comprises calculating a value of an uncertainty measure for the local data, wherein obtaining at least one of the consensus value and a parameter value derived from that consensus value comprises obtaining a value of an uncertainty measure for the obtained value, and wherein the step of using the obtained value as input in the process associated with operating the liquid treatment system is carried out on condition that the value of the uncertainty measure for the obtained value indicates a higher degree of certainty than the value of the uncertainty measure for the local data.

If the condition is not met, the local value comprised in or obtained on the basis of the local data is used as input in the process associated with operating the liquid treatment system.

In an embodiment of a method according to the first or according to the second aspect of the invention, the process associated with operating the liquid treatment system comprises at least one of:

adjusting at least one of the settings of the liquid treatment system; and
causing information representative of a condition of the liquid treatment system, e.g. a condition of the liquid treatment device, to be made accessible in perceptible form, e.g. via a user interface at a location of the liquid treatment system.

Settings include in particular settings affecting a degree of treatment or a volume of liquid treated in the liquid treatment system. Causing information representative of a condition of the liquid treatment system to be made accessible in perceptible form may comprise communicating the information to a device through which the user can access the information or that is arranged to pass on the information to a user, e.g. in a message transmitted to user equipment such as a smart phone running a dedicated application. The step may alternatively or additionally comprise displaying the information, either on a screen or simply by illuminating warning lights. The condition of the liquid treatment system may be simply which of a limited number of states the liquid treatment system is in, e.g. operating normally or in need of service. Customary are traffic-light indications comprising the states normal, imminently in need of service and in need of service.

In an example of this embodiment, the at least one setting comprises a setting of a flow regulating device for adjusting a rate of flow of liquid through the liquid treatment device.

Thus, the consensus value is the basis for adjusting the rate of flow of liquid through the liquid treatment device. In many cases, in particular where the degree of treatment depends on contact time of the liquid with a liquid treatment medium, the rate of flow will also determine how quickly the liquid treatment system is in need of servicing, e.g. reconditioning ion exchange resin, backwashing of a filtration membrane.

In a particular version of this example, each liquid treatment system defines a first flow path comprising a section extending through at least a section of the liquid treatment device and a second flow path of which at least a section extends separately from and in parallel to the section of the first flow path, and the setting of the flow regulating device determines a volumetric flow rate ratio of liquid flowing along the first flow path to liquid flowing along the second flow path.

This embodiment is of particular use where the treatment involves changing the concentration of certain salts in aqueous liquids. The liquid treatment itself is generally not variable, but the concentration in the blend of liquid that has been conducted along the first path and the liquid that has been conducted along the second path is variable.

In a particular example of any embodiment of a method according to the first or the second aspect of the invention in which the at least one setting comprises a setting of a flow regulating device for adjusting a rate of flow of liquid through the liquid treatment device, the parameter value derived from the consensus value comprises a target value of the setting.

In an embodiment of a method according to the first or the second aspect of the invention, the liquid treatment device comprises at least one of:

a liquid treatment medium for treatment of liquid in a diffusive process, e.g. for treatment of liquid by ion exchange; and
a liquid treatment medium soluble in the liquid.

In both cases, the liquid treatment medium requires replacement, replenishment or reconditioning after a certain volume of liquid has been treated, where this volume also depends on the concentration of components changeable by the liquid treatment device in the liquid to be treated.

In an embodiment of a method according to the first or the second aspect of the invention, the liquid treatment device comprises a bed of granular liquid treatment medium for treating liquid flowing through the bed.

In an embodiment of a method according to the first or the second aspect of the invention, each liquid treatment system comprises at least one replaceable liquid treatment cartridge and a head device, wherein the head device is in liquid communication with the source of liquid, wherein the head device and the replaceable liquid treatment cartridge comprise co-operating mechanical interfaces for releasably coupling the replaceable liquid treatment cartridge to the head device, and wherein the head device is configured to divert at least a fraction of liquid flowing from the source into the head device through the replaceable liquid treatment cartridge.

In this embodiment, the methods help ensure that replacement of the liquid treatment cartridges is synchronised across the set of liquid treatment systems.

In an embodiment of a method according to the first or the second aspect of the invention, the process associated with operating the liquid treatment system is a process of determining a treatment capacity value based at least on the consensus value.

The process thus yields as a result an indication of whether the treatment capacity of the liquid treatment system is exhausted or such exhaustion is imminent. This information is useful for scheduling maintenance, including automatic maintenance, where the methods of the invention ensure that the maintenance is synchronised. In particular, the treatment capacity values do not differ greatly for liquid treatment systems connected to the same source of liquid and operating in parallel.

In a particular example of this embodiment, each liquid treatment system comprises a system for determining volumetric flow data representing a volume of liquid flowing through the liquid treatment device in a period of time, and the treatment capacity value is further based on the volumetric flow data.

Thus, the treatment capacity value is based on actual use of the liquid treatment systems. This is more accurate than a mere indication of a period of time.

In an embodiment of a method according to the first or the second aspect of the invention, the process of determining the treatment capacity value is further based on a confidence value associated with the consensus value, wherein the process of determining the treatment capacity value comprises adjusting a previous treatment capacity value or re-calculating the treatment capacity value in dependence on the confidence value, wherein adjusting a previous treatment capacity value comprises calculating an amount by which to adjust the previous treatment capacity value based on the consensus value and volumetric flow data pertaining to a most recent one of multiple successive time windows, and wherein re-calculating the treatment capacity value comprises calculating the treatment capacity value based on the consensus value and volumetric flow data pertaining to the most recent and to at least one earlier one of the multiple successive time windows.

Thus, re-calculation is only carried out infrequently, namely when it is determined that there is a high likelihood that the consensus value was incorrect. This embodiment allows the consensus value initially to be based on local data from only a few of the liquid treatment systems in a set. Over time, as more sets of local data enter into the calculation of the consensus value, the confidence value can indicate a higher degree of confidence. In case the consensus value has changed as the degree of confidence has grown, the re-calculation is useful to ensure that the treatment capacity value accurately reflects the properties of the untreated liquid. It may also happen that newly arrived local data indicates a change of composition of the untreated liquid. In that case, the confidence value will indicate a lower degree of confidence until further new sets of local data result in more or less the same consensus value. This confirms that the change is really a change and not due to anomalous local data from one of the liquid treatment systems in the set. Until then, the re-calculation is repeated based on the most recent consensus value and volumetric flow data pertaining to the most recent and to those earlier time windows to which respective confidence values pertain that are below a threshold value.

The confidence value may represent the number of the multiple liquid treatment systems relative to a total number of liquid treatment systems in the set. Re-calculation may then be carried out for as long as the confidence value remains below a certain threshold value, e.g. 70 %. Re-calculating the treatment capacity value comprises calculating the treatment capacity value based on the most recent consensus value and volumetric flow data pertaining to the most recent and to those earlier ones of the multiple successive time windows for which the confidence values were below the threshold value. This helps ensure that the capacity value converges towards a likely correct value when initially based on local data from only a few liquid treatment systems or a default value not based on any local data, but, for example, a factory-set value.

According to a third aspect of the invention, there is provided a system for processing data obtained from a set of liquid treatment systems connected in parallel to a common source of liquid to be treated,

wherein the system comprises an interface for exchanging data with multiple ones of the liquid treatment systems in the set, and
wherein the system is configured to carry out a method of processing data obtained from a set of liquid treatment systems according to the first aspect of the invention.

According to a fourth aspect of the invention, there is provided a system for processing operational data of a liquid treatment, comprising:

at least an interface to a sensor for measuring at least one parameter for, at least in combination with setting information that is information on at least one setting of a liquid treatment system comprising the sensor, determining a value of a concentration measure that is a measure of a concentration of at least one component changeable by a liquid treatment device comprised the liquid treatment system in liquid flowing through the liquid treatment device; and
an interface for exchanging data with a further data processing system,
wherein the system is configured to carry out a method of controlling operation of a liquid treatment system according to the second aspect of the invention.

According to a fifth aspect of the invention, there is provided a computer program comprising instructions which, when the program is executed by a computer comprising an interface for exchanging data with multiple ones of a set of liquid treatment systems, cause the computer to carry out a method of processing data obtained from a set of liquid treatment systems according to the first aspect of the invention.

According to a sixth aspect of the invention, there is provided a computer program comprising instructions which, when the program is executed by a computer comprising at least (i) an interface to a sensor for measuring at least one parameter for, at least in combination with setting information that is information on at least one setting of a liquid treatment system comprising the sensor, determining a value of a concentration measure that is a measure of a concentration of at least one component changeable by a liquid treatment device comprised in the liquid treatment system in liquid flowing through the liquid treatment device, and (ii) an interface for exchanging data with a further data processing system, cause the computer to carry out a method of controlling operation of a liquid treatment system according to the second aspect of the invention.

The invention will be explained in further detail with reference to the accompanying drawings, in which:

Fig. 1    is a schematic diagram showing liquid treatment systems at two different sites that are in communication with a remote server system;

Fig. 2    is a schematic diagram of one of the liquid treatment systems;

Fig. 3    is a flow chart showing steps carried out by a liquid treatment system in a first method of operating the liquid treatment system;

Fig. 4    is a flow chart showing steps carried out by the remote server system or one of the liquid treatment systems a particular site in a method facilitating the operation of the liquid treatment system in accordance with the first method;

Fig. 5    is a flow chart showing steps carried out by a liquid treatment system in a second method of operating the liquid treatment system;

Fig. 6    is a flow chart showing steps carried out by the remote server system or one of the liquid treatment systems a particular site in a method facilitating the operation of the liquid treatment system in accordance with the second method;

Fig. 7    is a flow chart showing steps carried out by a liquid treatment system in a third method of operating the liquid treatment system; and

Fig. 8    is a flow chart showing steps carried out by the remote server system or one of the liquid treatment systems a particular site in a method facilitating the operation of the liquid treatment system in accordance with the third method.

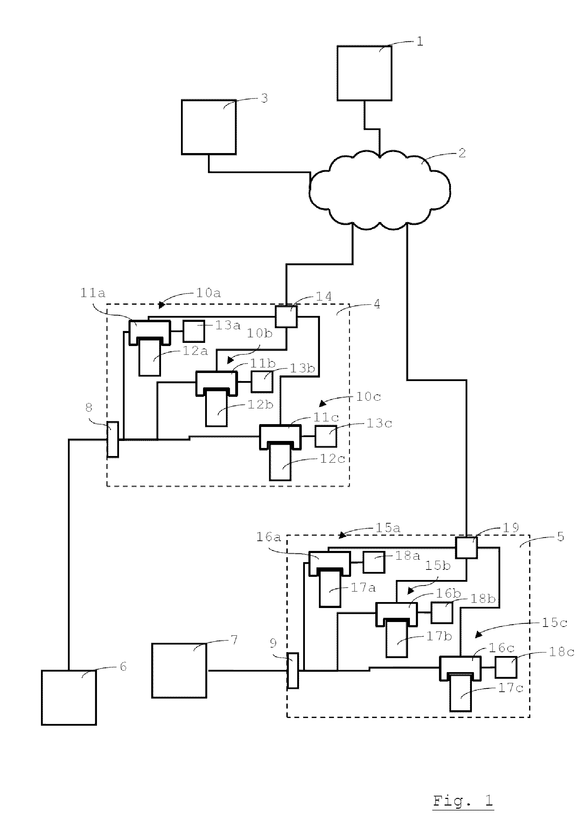

An example of a system of networked components for executing one or more methods to be described in the following comprises (Fig. 1) a remote server system 1 connected to a Wide Area Network (WAN) 2. The WAN 2 may comprise at least one of a cellular mobile network and a wired broadband network.

The remote server system 1 comprises a front-end component enabling a user to access the remote server system 1 using user equipment (UE) 3 e.g. a mobile data processing device such as a smart phone or tablet.

By way of example, first premises 4 and second premises 5 are supplied by a first source 6 and a second source 7 of water, in particular piped water. The first and second sources 6,7 are sources of pressurised water, e.g. mains drinking water. Other types of sources are possible, e.g. desalination plants or private water treatment plants. As an example, two treatment facilities, e.g. on one site or in a particular neighbourhood, may be connected to a common source of mains water and form the first and second sources 6,7.

As illustrated, a first point-of-entry connection 8 connecting to the first source 6 is installed at the first premises 4. A second point-of-entry connection 9 connecting to the second source 7 is installed at the second premises 5. The point-of-entry connections 8,9 may also be considered as sources in the context of the present disclosure.

A plurality of first liquid treatment systems 10a-c are arranged at the first premises 4 and connected to the first point-of-entry connection 8. Each of the first liquid treatment systems 10 comprises a first head device 11a-c and a first replaceable liquid treatment cartridge 12a-c.

The first liquid treatment systems 10a-c are arranged to supply treated liquid, in this example actually a mix of treated and untreated liquid, to a respective first appliance 13a-c. The first liquid treatment systems 10a-c may be capable of supplying the liquid to more than one first appliance 13a-c. Relevant appliances 13 include one or more of a steam cooker, laundry device, dish washer and devices for preparing and/or dispensing beverages, including hot or cold beverages or both. Cold beverage makers include devices comprising carbona-tors, batch or in-line, post-mix devices and the like. Hot beverage makers include devices for preparing beverages by extraction (tea or coffee makers, for example) and devices for preparing hot beverages by mixing with concentrate in powder or syrup form.

In the illustrated embodiment, the first head devices 11a-c are also arranged to function as nodes in a data communications network further including a first gateway device 14 that interconnects the network at the first premises 4 and the WAN 2.

In a similar manner, a set of second liquid treatment systems 15a-c connected to the second point-of-entry connection 9 for supply of liquid to be treated is provided at the second premises 5. Each of the multiple second liquid treatment systems 15a-c comprises a second head device 16a-c and a replaceable second liquid treatment cartridge 17a-c. Second appliances 18a-c are supplied by the second liquid treatment systems 15a-c. The second head devices 16 are arranged to function as nodes in a data communications network further including a second gateway device 19 interconnecting the data communications network at the second premises 5 and the WAN 2.

In alternative embodiments, one or more of the head devices 11a-c,16a-c may be connected directly to the WAN 2. They may in particular function as User Equipment in a (cellular) mobile data communications network. In that case, the gateway devices 14,19 may not be required.

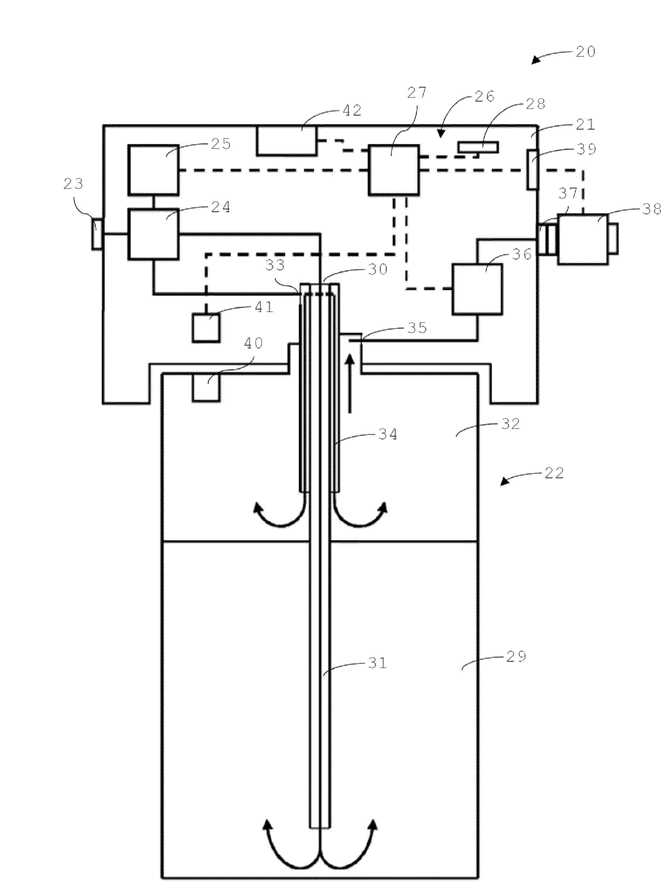

A representative example of a liquid treatment system 20 (Fig. 2) comprises a head device 21 and a replaceable liquid treatment cartridge 22. An inlet connector 23 is configured to connect the head device 21 to a pipeline in a network interconnecting the head device 21 and one of the point-of-entry connections 8,9.

Liquid supplied through the inlet connector 23 is conducted to a flow divider 24, from where a fraction continues along a first flow path and a remaining fraction is diverted to a second flow path. The illustrated flow divider 24 is a variable-ratio flow divider, e.g. as disclosed in WO 2009/101188 A1 or WO 2016/012537 A1. An actuator 25 controlled by a control device 26 is provided to change a setting of the variable-ratio flow divider 24 and thereby affect the volumetric flow rate ratio between the two fractions. The control device 26 comprises a data processing device 27 and memory 28. The two fractions join at a mixing location, as will be explained. The fraction led along the first path is subjected to a liquid treatment that the fraction led along the second path is not, or only to a lesser extent. The fraction led along the second path is therefore also referred to herein as the blending fraction x. The control device 26 may be programmed to relate positions of the actuator 25 to values of the blending fraction x. In a variant, flow sensors are provided to enable the control device 26 to control the blending fraction x.

The head device 21 and the liquid treatment cartridge 22 are provided with co-operating parts of a mechanical interface for releasably locking the liquid treatment cartridge 22 to the head device 21. The lock is established in a configuration in which at least the first, in the illustrated example also the second, flow path extends through the liquid treatment cartridge 22 and back to the head device 21. The configuration is a sealed configuration essentially preventing leakage of liquid at the interface.

The liquid treatment cartridge 22 illustrated comprises a first liquid treatment medium in the form of a first bed 29 of granular liquid treatment material or materials. The materials may in particular include one or more materials for the treatment of liquid by ion exchange. This includes at least cation exchange material, including weakly acidic or strongly acidic cation exchange material. The cation exchange material may, at least initially, be in the hydrogen form and thus arranged to reduce the carbonate hardness of water passing through the first bed 29. Carbonate hardness, also referred to as temporary hardness, is caused by the presence of calcium carbonate and magnesium carbonate in water. The concentration of these (dissolved) components therefore determines the value of a measure of the carbonate hardness. Some cation exchange material may initially be in the sodium or potassium form for buffering purposes. The ion exchange material may be a resin, for example.

The liquid treatment medium in the first bed 29 has a finite treatment capacity, which is exhausted as liquid flows through the first bed 29 and is treated. The rate of exhaustion depends on the volume of liquid treated and the concentration of components present in the liquid to be treated that is removed by the treatment. When the state of exhaustion has been reached or is imminent, this is signalled.

The first flow path extends through a first cartridge inlet 30 down an inner fall tube 31 into the first bed 29 and from there upwards into a second bed 32 of liquid treatment medium. The second flow path extends from a second cartridge inlet 33 through an outer fall tube 34 directly to the second bed 32. The second flow path thus bypasses the section of the first flow path extending through the first bed 29. The second bed 32 forms the mixing location.

The composition of the second bed 32 may differ from that of the first bed 29. The composition may be the same, since the extent to which the liquid is treated differs between the first and second flow paths. Generally, however, the liquid treatment medium in the second bed 32 will be configured to effect a different treatment. The treatment may still be a treatment comprising a diffusive process, e.g. sorption. The second bed 32 may, for example, contain activated carbon or a mineral-based material for the removal of one or more species of heavy metal.

The first and second flow paths extend through a common cartridge outlet 35 back to the head device 21. The liquid flows past a flow meter 36 to an outlet connector 37 and from there past a sensor device 38. The head device 21 comprises a sensor interface 39 to provide a signal from the sensor device 38 to the control device 26. In a variant, the sensor device 38 is replaced by one or more sensors internal to the head device 21.

The sensor device 38 comprises a sensor for measuring at least one parameter for, at least in combination with knowledge of the blending fraction x set by means of the flow divider 24, determining a value of a concentration measure that is a measure of a concentration of at least one component removable from liquid by the liquid treatment medium in the first bed 29. The parameter may in particular be one of electrical conductivity and electrical conductivity adjusted for deviations of ambient temperature from a reference temperature. In the latter case, the sensor device 38 comprises at least two sensors (one for the temperature and one for the electrical conductivity). The measured electrical conductivity values are converted into values that would have pertained if the temperature had been at a reference value, e.g. 25° C. This takes account of the fact that the electrical conductivity for a given concentration of conductive ion species varies with the temperature of the water. In a variant, the adjustment for temperature deviations is effected by the control device 26 on the basis of a signal from a temperature sensor in or connected to the head device 21.

In a variant, the parameter measured by the sensor device 38 is a parameter corresponding to the concentration measure, e.g. in case of a signal from an ion-selective conductivity sensor. This parameter, however, would still pertain to the mix of treated and untreated liquid, so that the setting of the flow divider 24 is needed to convert the parameter value to a concentration measure value pertaining to liquid to be treated. In another variant, the sensor device 38 is configured to measure acidity.

In the illustrated embodiment, the liquid treatment cartridge 22 is provided with a machine-readable token 40 and the head device 21 comprises a transceiver 41 for at least reading data from the machine-readable token 40. The machine-readable token 40 can be a bar code, RFID tag, QR code or the like.

The head device 21 also includes at least one interface 42 for communication with at least one of a user and an external device. The interface 42 may therefore comprise one or more devices for providing a user interface, e.g. a display device and/or at least one user input device. The interface 42 of the illustrated embodiment also comprises at least one network interface for connecting to the network also comprising one of the gateway devices 14,19. Thus, such a network interface may be an interface for connecting to a Wireless Local Area Network (WLAN). The interface 42 may alternatively or additionally comprise a wireless interface for short-range wireless communication, e.g. via a Personal Area Network (PAN). Thus, a suitably programmed UE 3 can provide the user interface.

One or both of the head device 21 and either the remote server system 1 or another head device 21 fulfilling a co-ordinating role are arranged to carry out a method of determining a value of a measure of a concentration of at least one component of which the concentration is changeable by the liquid treatment medium in the first bed 29, as well as a method of setting the blending fraction x. These methods are as disclosed in WO 2014/006128 A1, incorporated by reference in its entirety. For ease of reference, aspects of the disclosure are recapitulated here, using temporary hardness as the concentration measure and electrical conductivity or temperature-adjusted electrical conductivity as the measured parameter by way of example. For simplicity, the measured parameter is referred to as electrical conductivity, covering both alternatives (temperature-adjusted electrical conductivity and non-adjusted electrical conductivity).

The blending fraction $x$ is adjusted to or maintained at a first value $x_1$. The electrical conductivity $s(x_1)$ is then measured. The blending fraction $x$ is then adjusted to a second value $x_2$ different from the initial value $x_1$. A further measurement of the electrical conductivity $s(x_2)$ is then made. The temporary hardness $H$ of the untreated liquid is determined as follows:

$$H = \frac{s(x_2) - s(x_1)}{(x_2 - x_1) \cdot F}, \qquad\qquad (1)$$

where $F$ is a conversion factor. The conversion factor may be a constant - a reasonable value would be 30 μs/°dH - or a function of the blending fraction $x$.

The first value $x_1$ may be a value determined as appropriate to obtain a target value of the temporary hardness at the outlet of the liquid treatment system, here the outlet connector 37. The second value $x_2$ may be a default value, e.g. 0 % or 100 %.

In a variant, the first and second values $x_1, x_2$ are obtained by small adjustments from an initial value $x_0$, as follows:

$$x_1 = x_0 + \Delta x/2, \qquad\qquad (2)$$

$$x_2 = x_0 - \Delta x/2. \qquad\qquad (3)$$

In this variant, the initial value $x_0$ can be the value appropriate to obtain a target value of the temporary hardness at the outlet of the liquid treatment system, here the outlet connector 37, in view of a previously determined value of the temporary hardness $H$ of the untreated liquid. The deviation $\Delta x$ is small enough to maintain suitability of the liquid supplied at the outlet, here the outlet connector 37, for the intended purpose. Furthermore, a different entity from the control device 26 can perform the hardness determination on the basis of only three values: the initial value $x_0$ or the equivalent setting of the flow divider 24 and the two measured electrical conductivity values $s(x_1), s(x_2)$. The same is of course the case in the variant in which the second value $x_2$ is a pre-determined value such as 100 %.

It is possible to associate a confidence value with the calculated value of the temporary hardness. For example, if the second value $x_2$ of the blending fraction is 100 %, it is possible to calculate an expected mid-point of a band of electrical conductivity values $s(x)$ measured by the sensor device 38. This can be compared with an actual mid-point determined over time. The absolute value of the difference serves as a confidence value.

The blending fraction value $x$ and the signal from the flow meter 36 are used to determine the volume of liquid flowing through the first bed 29. This value, weighted by the temporary hardness $H$ is integrated as part of a process of calculating at least one of the remaining and used treatment capacity of the liquid treatment medium in the first bed 29. Imminent exhaustion is determined when this running value approaches the initially available treatment capacity, respectively zero.

It will often be the case that the average rate of consumption of water by the appliances 13a-c at e.g. the first premises 4 is the same. They may be steam cookers or coffee machines operated side-by-side in the same fast-food restaurant or café, for example. For such scenarios, methods are provided to synchronise the signalling of imminent exhaustion. The liquid treatment cartridges 12a-c can then be replaced at the same time. Furthermore, hardness values $H$ on which the capacity determinations are based are relatively accurate, even if, as here, the blending fraction $x$ is inferred from the setting of the flow divider 24, rather than by measuring the rates of flow along both the first flow path and the second flow path. Compared with an alternative based on averages of multiple measurements carried out sequentially over time, fewer adjustments of the flow divider 24 are required to achieve relatively high accuracy within available tolerance ranges of the sensor device 38 and the components of the system for adjusting the blending ratio $x$.

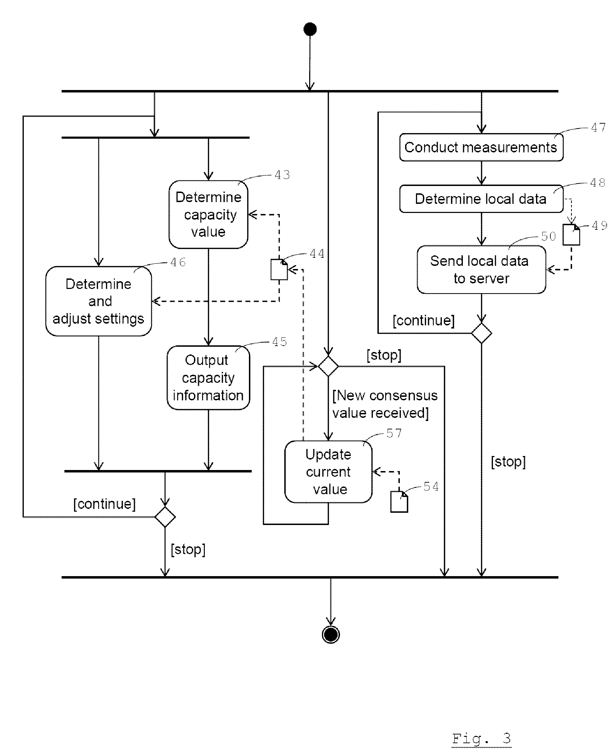

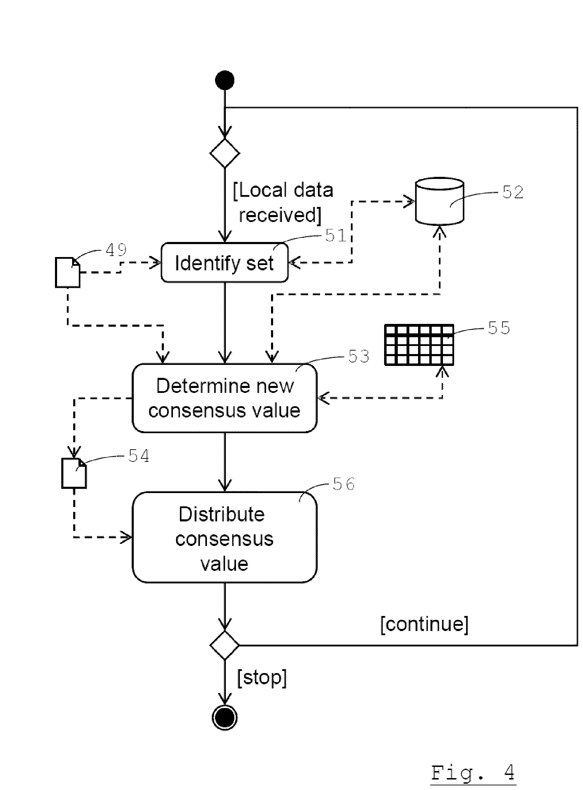

In a first implementation, a method of operating one of the liquid treatment systems 10a-c,15a-c,20 (Fig. 3) and a method of facilitating operation of the liquid treatment systems 10a-c,15a-c,20 (Fig. 4) are structured such as to minimise data transfer.

For simplicity, the method of facilitating operation of the liquid treatment systems 10a-c,15a-c,20 (Fig. 4) will be described on the assumption that the remote server system 1 carries out this method. It is equally possible to designate one of the head devices 11a-c of the first liquid treatment systems 10a-c as a master device for all the first liquid treatment systems 10a-c at the first premises 4 and to do the same for the second liquid treatment systems 15a-c at the second premises 5. The head device 11a-c,16a-c,21 designated as the master device will then carry out the method of facilitating the operation of the liquid treatment systems 10a-c,15a-c at the same premises 4,5.

Turning to the steps carried out by a liquid treatment system 20 when not fulfilling a role as a master device (Fig. 3), the data processing device 27 is configured to keep (step 43) a running integral of the volume of liquid flowing through the first bed 29, weighted by a current value 44 of the temporary hardness. Initially, this current value 44 may be a default value, but the current value 44 is updated over time and thus variable. The result of this step 43 is at least one of a value of the capacity used since the last replacement of the liquid treatment cartridge 22 or of the capacity remaining. In an embodiment, the liquid treatment system 20 merely deducts from the value of the remaining capacity or adds to the value of the used capacity a value corresponding to the volume flowing in a most recent time window, weighted by the then valid current value 44. In a variant, this is made conditional on a confidence value associated with the current value 44 being above a threshold value. If the value is below the threshold value, on the other hand, the capacity value is re-calculated for all preceding time windows in which the confidence value was below the threshold value. Re-calculation comprises weighting the volumes flowing in these preceding time windows by the latest current value 44. This ensures that a probably incorrect current value 44 does not have a lasting effect on the calculated capacity value.

Capacity information is output (step 45) next. The capacity information may correspond to the capacity value determined in the preceding step 43 or be based thereon. Common practice, for example, is to define three states, corresponding to normal, imminent exhaustion and exhaustion. Outputting capacity information may comprise making capacity information accessible in perceptible form via a user interface comprised in the interface 42 of the head device 21 or at a location of the liquid treatment system 20. In the latter case, data can be transmitted to a device providing a user interface at the premises at which the liquid treatment system 20 is located. Alternatively or additionally, the capacity information may be transmitted to the remote server system 1. The remote server system 1 may in turn forward the capacity information to the UE 3 of a user registered as being associated with the liquid treatment system 20. Alternatively or additionally, the remote server system 1 may update capacity information held for the liquid treatment system 20 in a database 52 (Fig. 4) accessible to that user using the UE 3.

In parallel, the data processing device 27 uses the current value 44 of the temporary hardness to determine an appropriate setting and adjust (step 46) the setting of the flow divider 24 such as to supply water with a target value of the temporary hardness.

These steps 43,45,46 are repeated until operation of the liquid treatment system 20 is halted. The trigger to continue can be elapse of a pre-determined time period, updating of the current value 44, the passing of a pre-determined volume of water, the reaching of a particular re-occurring point in time (e.g. a particular weekly or daily point in time), a combination of these triggers or the like.

The liquid treatment system 20 is also configured to conduct (step 47) measurements of the electrical conductivity $s(x)$ to obtain the values $s(x_1),s(x_2)$ at the first and second values $x_1,x_2$ of the blending fraction. The parameter values $s(x_1),s(x_2)$ and, if needed, one or both of the first and second values $x_1,x_2$ are used in a step 48 of determining a set of local data 49. The local data 49 are sent (step 50) to the remote server system 1.

In one implementation, the local data 49 comprise the parameter values $s(x_1),s(x_2)$ and those of the first and second values $x_1,x_2$ needed to determine a local value of the temporary hardness according to equation (1). Alternatively, the data processing device 27 in the head device 21 calculates the local value of the temporary hardness and the local data 49 comprises the local value of the temporary hardness. Here, a local value of the concentration measure, in this example a local value of the temporary hardness, is a value based on at least one value of the parameter, in this example, electrical conductivity, where the at least one values are measured exclusively at a liquid treatment system 20 with which that local value is associated.

The steps 47,48,50 just described are repeated until operation of the liquid treatment system 20 is halted. The trigger to continue can be elapse of a pre-determined time period, updating of the current value 44, the passing of a pre-determined volume of water, the reaching of a particular re-occurring point in time (e.g. a particular weekly or daily point in time), a combination of these triggers or the like. The trigger need not be the same as the one causing a repeat of the other steps 43,45,46 that are carried out iteratively. The pre-determined volumes or time periods, if used as a trigger, may differ, for example.

The remote server system 1 receives the local data and, upon receipt, identifies (step 51) a set of liquid treatment systems to which the liquid treatment system 20 that has sent the local data belongs. In the example used here, this means determining whether the liquid treatment system 20 is one of the first liquid treatment systems 10a-c or one of the second liquid treatment systems 15a-c.

In one implementation, each head device 11a-c,16a-c,21 is associated with a unique identifier (e.g. a serial number) in the database 52. The database 52 is accessible to a user, e.g. using the UE 3, to enable the user to associate tagging data with the head devices 11a-c,16a-c,21 of the liquid treatment systems 10a-c,15a-c,20 operated by that user. The tagging data can be communicated to the head device 21, which then sends the tagging data with the local data 49. Alternatively, the local data 49 can be sent with identifying information forming a key into the database 52 allowing the tagging data to be retrieved from the database 52. Members of a set of liquid treatment systems 10a-c,15a-c,20 are identified based on correspondence of tagging data.

It is also possible for the liquid treatment system 20, e.g. the head device 21, to comprise a positioning device for determining a geographical location of the liquid treatment system 20. Geographical data for determining a geographical location of the liquid treatment system 20 can then be sent with the local data 49 or in a separate configuration step or the like. The remote server system 1 will be configured to identify members of a set of liquid treatment systems 10a-c,15a-c,20 based on proximity to each other.

It is also possible for the members of a set to be identified by detecting their presence as nodes in a communication network, e.g. as nodes in a LAN or PAN at one of the first and second premises 4,5. Alternatively, the set to which they belong can be identified on the basis of their address in such a network or, in case they are present as nodes in the WAN 2, their address in the WAN 2. As yet another alternative, the address of the gateway device 14,19 from which the local data 49 are received can be used to identify members of a set of liquid treatment systems 10a-c,15a-c,20.

As mentioned, if the local data 49 do not already comprise a local value of the temporary hardness calculated by the head device 21, the remote server system 1 will proceed to calculate that local value as part of a step 53 of determining a new consensus value 54 on the basis of the local data just received and local values obtained for other members of the set identified, which local values may be retrieved from the database 52 or comprised in a separate array 55 of data maintained for the set. The array 55 comprises for each liquid treatment system in the set, the latest available local data or local value based on the latest available local data, optionally in association with the confidence value mentioned above.

Determining a new consensus value 54 may comprise calculating an average value, e.g. a mean or median value, optionally subsequent to elimination of outliers. As an example, the median value may be taken in case of an odd number of members and the mean of the median two values in case of an even number of members. Outliers can be values deviating by more than a certain multiple of a standard deviation from the median value. It is also possible to use an artificial neural network (ANN). Such an ANN may be trained on a large number of sets of local data in the form of parameter values $s(x_1), s(x_2)$ and any required values of the blending fraction $x_1, x_2$ or equivalently settings of the flow divider 24 checked against hardness values obtained using samples analysed using more accurate laboratory equipment, for example.

If the local data 49 include the confidence value mentioned above and each local value is associated with a respective confidence value, these confidence values can be used to discard, ignore or weight the local values associated with the members of the set of liquid treatment systems 10a-c,15a-c,20 when determining the consensus value 54.

The remote server system 1 distributes the new consensus value 54 (step 56). In one implementation, the new consensus value 54 is sent to each member of the set. In another implementation, only those liquid treatment systems of which the local value in the array 55 deviates by less than a certain amount (absolute or relative), are sent the new consensus value 54. This takes account of the possibility that certain liquid treatment systems may have been incorrectly identified as members of the set, e.g. because a user has not tagged them correctly.

In one implementation, the consensus value 54 is associated with a confidence value that is sent with the consensus value 54. The confidence value depends on the number of sets of local data 49 used actually to calculate the consensus value 54, e.g. increasing from 10 % to 70 %. Here, the confidence value represents the number of sets of local data 49 used to calculate the consensus value 54 relative to the total number of liquid treatment systems in the set.

In this way, when there are many outliers, which are removed, the confidence value decreases. This serves as an indication that that certain liquid treatment systems may have been incorrectly identified as members of the set, so that the consensus value 54 may not be appropriate for them. It is further possible to weight the set of local data 49, such that the confidence value increases by more when more recently received sets of local data 49 are used than when older set of local data 49 are used. This takes account of the possibility that the composition of the untreated liquid may have changed.

The remote server system 1 continues to wait for the arrival of new local data until operation of the remote server system 1 is halted.

The head device 11, upon receiving the consensus value 54 (Fig. 3), replaces (step 57) the current value 44 by the consensus value 54. Where the head device 11 has previously calculated the local value, the replacement may be conditional upon the consensus value deviating by less than a certain amount (absolute or relative) from that calculated local value. Where the head device 11 has previously calculated the local value together with a confidence value and has also received a consensus value together with a confidence value, the replacement may be conditional upon the confidence value received with the consensus value indicating a higher level of confidence than the confidence value calculated for the calculated local value of the measure of the concentration of components contributing to temporary hardness.

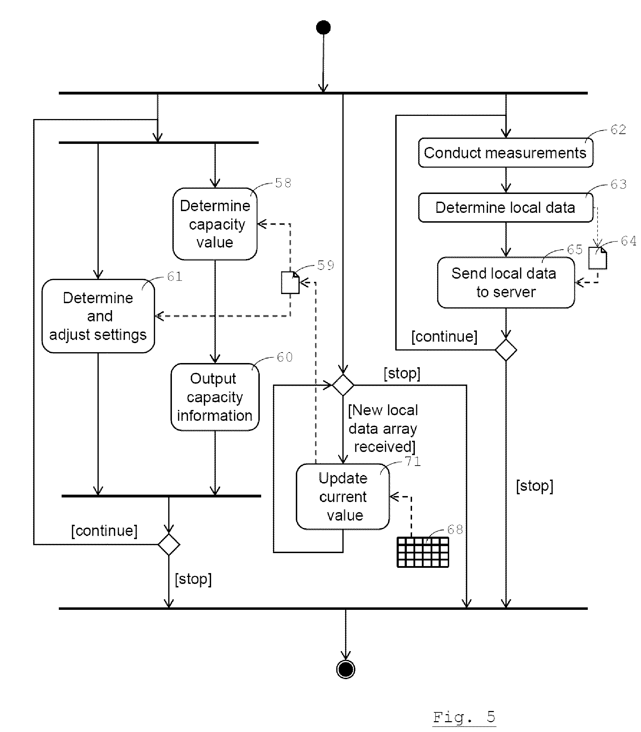

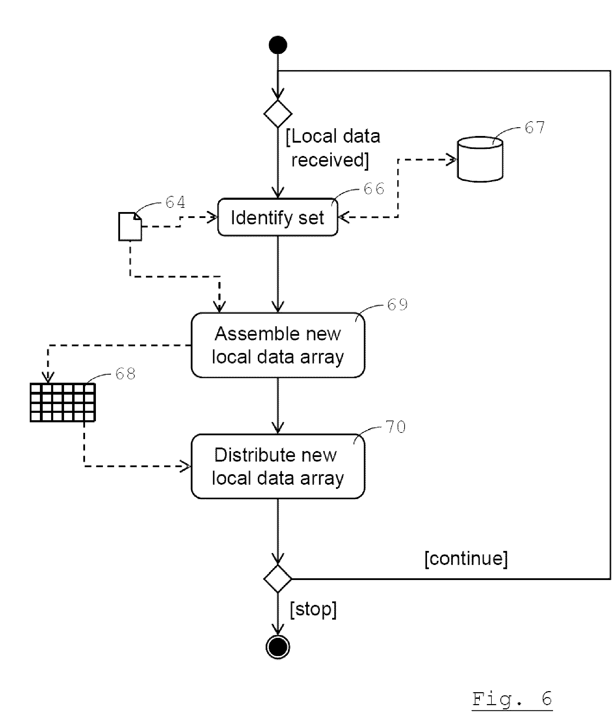

In a second implementation, a method of operating one of the liquid treatment systems 10a-c,15a-c,20 and a method of facilitating operation of the liquid treatment system 10a-c,15a-c,20 are combined into a method (Fig. 5) carried out by the head device 21 of the liquid treatment system 20 with only minimal involvement (Fig.6) of the remote server system 1.

As in the first implementation, it is possible to designate one of the head devices 11a-c of the first liquid treatment systems 10a-c as a master device for all the first liquid treatment systems 10a-c at the first premises 4 and to do the same for the second liquid treatment systems 15a-c at the second premises 5. The head device 11a-c,16a-c,21 designated as the master device will then carry out those steps (Fig. 6) that the following explanation describes as being carried out by the remote server system 1.

As is the case in the first implementation, the data processing device 27 of the head device 21 is configured to keep (step 58) a running integral of the volume of liquid flowing through the first bed 29, weighted by a current value 59 of the temporary hardness. Initially, this current value 59 may be a default value, but the current value 59 is updated over time and thus variable. The result of this step 58 is at least one of a value of the capacity used since the last replacement of the liquid treatment cartridge 22 or of the capacity remaining. As in the first implementation, an implementation is possible in which the data processing device 27 of the head device 21 merely deducts from the value of the remaining capacity or adds to the value of the used capacity a value corresponding to the volume flowing in a most recent time window, weighted by the then valid current value 59. As in the first implementation, the mere incrementation or deduction may be conditional on a confidence value associated with the current value 59 being above a threshold value. If the value is below the threshold value, on the other hand, the capacity value is re-calculated for all preceding time windows in which the confidence value was below the threshold value. Re-calculation comprises weighting the volumes flowing in these preceding time windows by the latest current value 59. This ensures that a probably incorrect current value 59 does not have a lasting effect on the calculated capacity value.

Capacity information is output (step 60) next. The capacity information may correspond to the capacity value determined in the preceding step 58 or be based thereon. Outputting capacity information may comprise making capacity information accessible in perceptible form via a user interface of the head device 21 or at a location of the liquid treatment system 20. In the latter case, data can be transmitted to a device providing a user interface at the premises at which the liquid treatment system 20 is located. Alternatively or additionally, the capacity information may be transmitted to the remote server system 1. The remote server system 1 may in turn forward the capacity information to the UE 3 of a user registered as being associated with the liquid treatment system 20. Alternatively or additionally, the remote server system 1 may update capacity information held for the liquid treatment system 20 in a database 67 (Fig. 6) accessible to that user using the UE 3.

In parallel, the data processing device 27 uses the current value 59 of the temporary hardness to determine an appropriate setting and adjust (step 61) the setting of the flow divider 24 such as to supply water with a target value of the temporary hardness.

These steps 58,60,61 are repeated until operation of the liquid treatment system 20 is halted. The trigger to continue can be elapse of a pre-determined time period, updating of the current value 59, the passing of a pre-determined volume of water, the reaching of a particular re-occurring point in time (e.g. a particular weekly or daily point in time), a combination of these triggers or the like.

The liquid treatment system 20 is also configured to conduct (step 62) measurements of the electrical conductivity $s(x)$ to obtain the values $s(x_1),s(x_2)$ at the first and second values $x_1,x_2$ of the blending fraction. The parameter values $s(x_1),s(x_2)$ and, if needed, one or both of the first and second values $x_1,x_2$ are used in a step 63 of determining a set of local data 64. The local data 64 are sent (step 65) to the remote server system 1.

In one implementation, the local data 64 comprise the parameter values $s(x_1),s(x_2)$ and those of the first and second values $x_1,x_2$ needed to determine a local value of the temporary hardness according to equation (1). Alternatively, the data processing device 27 in the head device 21 calculates the local value of the temporary hardness and the local data 64 comprises the local value of the temporary hardness. Again, a local value of the concentration measure, in this example a local value of the temporary hardness, is a value based on at least one value of the parameter, in this example, electrical conductivity, where the at least one values are measured exclusively at a liquid treatment system 20 with which that local value is associated.

The steps 62,63,65 just described are repeated until operation of the liquid treatment system 20 is halted. The trigger to continue can be elapse of a pre-determined time period, updating of the current value 59, the passing of a pre-determined volume of water, the reaching of a particular re-occurring point in time (e.g. a particular weekly or daily point in time), a combination of these triggers or the like. The trigger need not be the same as the one causing a repeat of the other steps 58,60,61 that are carried out iteratively. The pre-determined volumes or time periods, if used as a trigger, may differ, for example.

The remote server system 1 receives the local data and, upon receipt, identifies (step 66) a set of liquid treatment systems to which the liquid treatment system 20 that has sent the local data belongs. In the example used here, this means determining whether the liquid treatment system 20 is one of the first liquid treatment systems 10a-c or one of the second liquid treatment systems 15a-c.

Again, each head device 11a-c,16a-c,21 may be associated with a unique identifier (e.g. a serial number) in the database 67. The database 67 is accessible to a user, e.g. using the UE 3, to enable the user to associate tagging data with the head devices 11a-c,16a-c,21 of the liquid treatment systems 10a-c,15a-c,20 operated by that user. The tagging data can be communicated to the head device 21, which then sends the tagging data with the local data 64. Alternatively, the local data 64 can be sent with identifying information forming a key into the database 67 allowing the tagging data to be retrieved from the database 67. Members of a set of liquid treatment systems 10a-c,15a-c,20 are identified based on correspondence of tagging data.

The liquid treatment system 20, e.g. the head device 21 may alternatively or additionally comprise a positioning device for determining a geographical location of the liquid treatment system 20. Geographical data for determining a geographical location of the liquid treatment system 20 can then be sent with the local data 64 or in a separate configuration step or the like. The remote server system 1 will be configured to identify members of a set of liquid treatment systems 10a-c,15a-c,20 based on proximity to each other.

The members of a set may be identified by detecting their presence as nodes in a communication network, e.g. as nodes in a LAN or PAN at one of the first and second premises 4,5. Alternatively, the set to which they belong can be identified on the basis of their address in such a network or, in case they are present as nodes in the WAN 2, their address in the WAN 2. As yet another alternative, the address of the gateway device 14,19 from which the local data 64 are received can be used to identify members of a set of liquid treatment systems 10a-c,15a-c,20.

In the second implementation, the remote server system 1 merely maintains or assembles the array 68 of local data 64 associated with the members of each set. Whenever new local data 64 is received, the array 68 is updated (step 69) with the new local data 64. The array 68 thus comprises for each liquid treatment system in the set, the latest available local data or local value based on the latest available local data, optionally in association with the confidence value mentioned above.

The remote server system 1 merely distributes (step 70) the array 68 to the liquid treatment systems 10a-c,15a-c,20 in the set. The role of the remote server system 1 is thereby concluded. The remote server system 1 merely waits for the next local data 64 to arrive from one of the liquid treatment systems 10a-c,15a-c,20. In a variant, the remote server system 1 does not re-send the entire array 68. Instead, the liquid treatment systems 10a-c,15a-c,20 maintain the array and the remote server system 1 sends new values with an identification of the liquid treatment system 10a-c,15a-c,20 to which the respective values pertain. The liquid treatment systems 10a-c,15a-c,20 can thus update their copy of the array 68 themselves.

When the liquid treatment system 20 receives the array 68, the liquid treatment system 20 proceeds to update (step 71) the current value 59. This step 71 comprises determining a consensus value based on the local data in the array 68.

Determining the consensus value may comprise calculating an average value, e.g. a mean or median value, optionally subsequent to elimination of outliers. As an example, the median value may be taken in case of an odd number of members and the mean of the median two values in case of an even number of members. Outliers can be values deviating by more than a certain multiple of a standard deviation from the median value. It is also possible to use an artificial neural network (ANN). Such an ANN may be trained on a large number of sets of local data in the form of parameter values $s(x_1), s(x_2)$ and any required values of the blending fraction $x_1, x_2$ or equivalently settings of the flow divider 24 checked against hardness values obtained using samples analysed using more accurate laboratory equipment, for example.

If the array 68 associates each local value with a respective confidence value, these confidence values can be used to discard, ignore or weight the local values associated with the members of the set of liquid treatment systems 10a-c,15a-c,20 when determining the consensus value.

In one implementation, the consensus value only replaces the previous current value 59 if the consensus value deviates by less than a certain amount (absolute or relative) from the local value calculated (step 63) on the basis of only the measurements conducted (step 62) locally at the liquid treatment system 20. This takes account of the possibility the array 68 contains local values pertaining to liquid treatment systems that have been incorrectly identified as members of the set, e.g. because a user has not tagged them correctly. It also takes account of the possibility that the liquid treatment system 20 has been identified as a member of the wrong set.

In one implementation, a confidence value is calculated for the consensus value. Replacement of the previous current value 59 can then be made conditional upon the confidence value indicating more than a certain degree of confidence. It is also possible to choose between the consensus value and the local value calculated (step 63) on the basis of only measurements conducted (step 62) locally at the liquid treatment system 20 in dependence on which one of the two values is associated with a higher degree of confidence according to the calculated associated confidence values. The chosen value becomes the new current value 59.

A variant of the second implementation is conceivable in which the array 68 is maintained locally at each of the liquid treatment systems 10a-c,15a-c,20 and they broadcast or multicast new values of local data 64 to all other liquid treatment systems 10a-c,15a-c,20 in the same Local Area Network or Personal Area Network. The remote server system 1 can then be dispensed with altogether, the amount of data exchanged is relatively limited and identification of members of a set is relatively straightforward. The liquid treatment systems operate relatively autonomously.

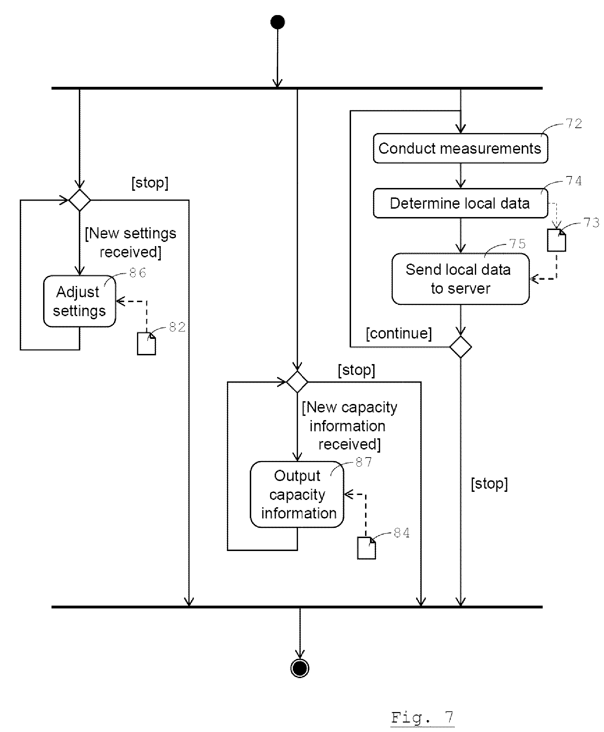

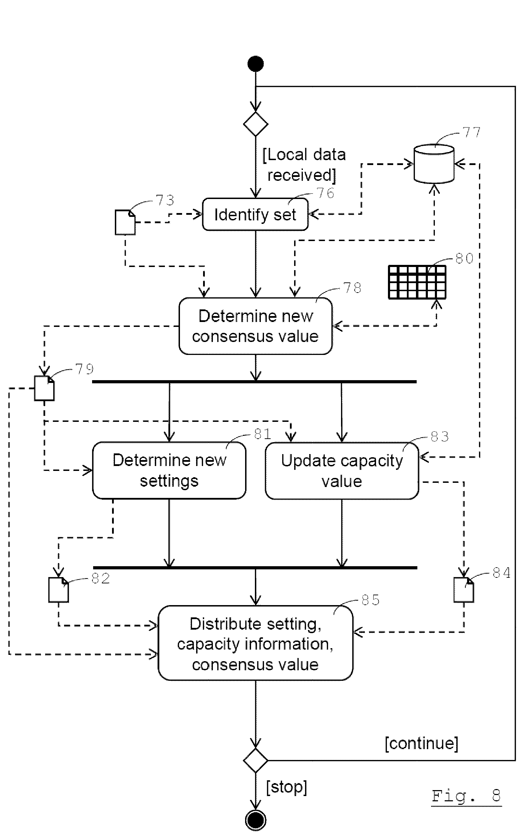

The opposite is the case for a third implementation, in which the functionality (Fig. 7) of the liquid treatment system 20 is relatively limited, whereas the remote server system 1 or a liquid treatment system 20 designated as master carries out most of a method of operating each liquid treatment system 20 as well as a method of facilitating operation of each liquid treatment system 20 (Fig. 8).

The liquid treatment system 20 is again configured to conduct (step 72) measurements of the electrical conductivity $s(x)$ to obtain the values $s(x_1),s(x_2)$ at the first and second values $x_1,x_2$ of the blending fraction. The parameter values $s(x_1),s(x_2)$ and, if needed, one or both of the first and second values $x_1,x_2$ are used in a step 74 of determining a set of local data 73.

In one implementation, the local data 73 comprise the parameter values $s(x_1),s(x_2)$ and those of the first and second values $x_1,x_2$ needed to determine a local value of the temporary hardness according to equation (1). Alternatively, the data processing device 27 in the head device 21 calculates the local value of the temporary hardness and the local data 73 comprises the local value of the temporary hardness. Again, a local value of the concentration measure, in this example a local value of the temporary hardness, is a value based on at least one value of the parameter, in this example, electrical conductivity, where the at least one values are measured exclusively at a liquid treatment system 20 with which that local value is associated.

The local data 73 also includes a value representative of the volume of water passed through the first bed 29 in a certain period of time, e.g. the period since the last iteration of these steps 72,74. It is also possible to sub-divide such period into smaller intervals and to associate a value of the volumetric flow data with each interval. The local data 73 are sent (step 75) to the remote server system 1.

The steps 72,74,75 just described are repeated until operation of the liquid treatment system 20 is halted. The trigger to continue can be elapse of a pre-determined time period, the passing of a pre-determined volume of water, the reaching of a particular re-occurring point in time (e.g. a particular weekly or daily point in time), a combination of these triggers or the like.

The remote server system 1 receives the local data and, upon receipt, identifies (step 76) a set of liquid treatment systems to which the liquid treatment system 20 that has sent the local data belongs. In the example used here, this means determining whether the liquid treatment system 20 is one of the first liquid treatment systems 10a-c or one of the second liquid treatment systems 15a-c.

Again, each head device 11a-c,16a-c,21 may be associated with a unique identifier (e.g. a serial number) in the database 77. The database 77 is accessible to a user, e.g. using the UE 3, to enable the user to associate tagging data with the head devices 11a-c,16a-c,21 of the liquid treatment systems 10a-c,15a-c,20 operated by that user. The tagging data can be communicated to the head device 21, which then sends the tagging data with the local data 73. Alternatively, the local data 73 can be sent with identifying information forming a key into the database 77 allowing the tagging data to be retrieved from the database 77. Members of a set of liquid treatment systems 10a-c,15a-c,20 are identified based on correspondence of tagging data.

The liquid treatment system 20, e.g. the head device 21 may alternatively or additionally comprise a positioning device for determining a geographical location of the liquid treatment system 20. Geographical data for determining a geographical location of the liquid treatment system 20 can then be sent with the local data 73 or in a separate configuration step or the like. The remote server system 1 will be configured to identify members of a set of liquid treatment systems 10a-c,15a-c,20 based on proximity to each other.

The members of a set may be identified by detecting their presence as nodes in a communication network, e.g. as nodes in a LAN or PAN at one of the first and second premises 4,5. Alternatively, the set to which they belong can be identified on the basis of their address in such a network or, in case they are present as nodes in the WAN 2, their address in the WAN 2. As yet another alternative, the address of the gateway device 14,19 from which the local data 73 are received can be used to identify members of a set of liquid treatment systems 10a-c,15a-c,20.

If the local data 73 comprise the parameter values $s(x_1),s(x_2)$ and those of the first and second values $x_1,x_2$ needed to determine a local value of the temporary hardness according to equation (1), the remote server system 1 will proceed to calculate that local value and optionally a confidence value for that local value as part of a step 78 of determining a new consensus value 79 on the basis of the local data just received and local values obtained for other members of the set identified. These local values may be retrieved from the database 77 or comprised in a separate array 80 of data maintained for the set. The array 80 comprises for each liquid treatment system in the set, the latest available local data or local value based on the latest available local data, optionally in association with the confidence value mentioned above.

If the local data 73 received already comprise a calculated local value, with or without a confidence value, it suffices for the remote server system 1 to obtain an updated array 80 and calculate the consensus value 79.

Determining a new consensus value 79 may comprise calculating an average value, e.g. a mean or median value, optionally subsequent to elimination of outliers. As an example, the median value may be taken in case of an odd number of members and the mean of the median two values in case of an even number of members. Outliers can be values deviating by more than a certain multiple of a standard deviation from the median value. It is also possible to use an artificial neural network (ANN). Such an ANN may be trained on a large number of sets of local data in the form of parameter values $s(x_1),s(x_2)$ and any required values of the blending fraction $x_1,x_2$ or equivalently settings of the flow divider 24 checked against hardness values obtained using samples analysed using more accurate laboratory equipment, for example.

If confidence values are associated with the local values, these confidence values can be used to discard, ignore or weight the local values associated with the members of the set of liquid treatment systems 10a-c,15a-c,20 when determining the consensus value 79.

In the third implementation, the remote server system 1 calculates (step 81) the appropriate setting 82 for the flow divider 24 based on the new consensus value 79. The remote server system 1 also calculates (step 83) an updated capacity value 84 for the liquid treatment system 20. Use of the new consensus value 79 in either or both steps 81,83 may be conditional upon the consensus value deviating by less than a certain amount (absolute or relative) from the local value calculated for the liquid treatment system 20 concerned or on a confidence value calculated for the new consensus value 79 indicating more than a certain level of confidence or a higher level of confidence than a confidence value associated with the local value for that liquid treatment system 20. Otherwise, the local value is used. As in the first and second implementations, an implementation is possible in which the remote server system 1 merely deducts from a capacity value 84 representing the remaining capacity or adds to a capacity value 84 representing the used capacity a value corresponding to the volume flowing in a most recent time window, weighted by the then valid consensus value 79. As in the other implementations, the mere incrementation or deduction may be conditional on a confidence value associated with the current consensus value 79 being above a threshold value. If the value is below the threshold value, on the other hand, the capacity value 84 is re-calculated for all preceding time windows in which the confidence value was below the threshold value. The volume flowing in these preceding time windows is weighted by the current consensus value 79, instead of by the then valid consensus values 79. Here, the confidence value may represent the number of sets of local data 73 used to calculate the consensus value 79 relative to the total number of liquid treatment systems in the set. The threshold value may thus be a percentage value or a value between 0 and 1.

The target value of the measure of the concentration of components removable from the liquid, in this case the target value of the temporary hardness, may be obtained from the database 77. For example, a user may set the target value using the UE 3. The target value may also be read from the machine-readable token 40 or set locally using the interface 42 and communicated to the remote server system 1.

The setting 82, the capacity value 84 or equivalent capacity information, and the consensus value 79 are sent (step 85) to the liquid treatment system 20. This is done individually for each liquid treatment system 10a-c,15a-c,20 in the set, optionally conditional upon there having been a change in one or more of these values for the liquid treatment system 20.

The remote server system 1 then continues to await the arrival of new local data 73.

The liquid treatment system 20, upon receiving the new setting 82, adjusts (step 86) the flow divider 24.

The liquid treatment system 20, upon receipt of the capacity information, e.g. the capacity value 84, outputs (step 87) the capacity information or information based thereon. Outputting capacity information may comprise making capacity information accessible in perceptible form via a user interface of the head device 21 or at a location of the liquid treatment system 20. In the latter case, data can be transmitted to a device providing a user interface at the premises at which the liquid treatment system 20 is located.

The output may in particular be in accordance with a traffic-light system, i.e. one value for normal operation, one value if exhaustion is imminent and one value if a state of exhaustion has been reached. By making use of consensus values 54,79, the capacity information that is output for the first liquid treatment systems 10a-c will generally correspond. This is all the more the case if their respective settings are based on the same consensus value 54,79. The same is the case for the set of second liquid treatment systems 15a-c.

The invention is not limited to the examples described above, which may be varied within the scope of the accompanying claims. For example, although the example of different premises 4,5 has been used here, it is not decisive whether the liquid treatment systems supplied by a common source 6,7 are located on the same or different premises as other liquid treatment systems supplied by a common, but different, source. The liquid treatment systems connected to a common source will generally not, however, be separated from the common source by any liquid treatment device arranged to effect a treatment on liquid flowing through the liquid treatment device that is liable to affect the concentration of the component changeable by the liquid treatment device of the liquid treatment systems connected to the common source.

Although the example of a liquid treatment system that produces a blend of treated and untreated liquid has been used here, the principles set out apply equally to a use case in which replacement of the liquid treatment cartridge 22 is occasioned by exhaustion of liquid treatment medium in the second bed 32 so that the parameter value obtained using the sensor device 38 can be converted into a value of the concentration measure regardless of the setting of the flow divider 24.

**List of reference numerals**

1 - Remote server system
2 - WAN
3 - UE

| | |
|---|---|
| 4 - | 1st premises |
| 5 - | 2nd premises |
| 6 - | 1st source |
| 7 - | 2nd source |
| 8 - | 1st point-of-entry connection |
| 9 - | 2nd point-of-entry connection |
| 10a-c - | 1st liquid treatment systems |
| 11a-c - | 1st head devices |
| 12a-c - | 1st liquid treatment cartridges |
| 13a-c - | 1st appliances |
| 14 - | 1st gateway device |
| 15a-c - | 2nd liquid treatment systems |
| 16a-c - | 2nd head devices |
| 17a-c - | 2nd liquid treatment cartridge |
| 18a-c - | 2nd appliances |
| 19 - | 2nd gateway device |
| 20 - | Liquid treatment system |
| 21 - | Head device |
| 22 - | Liquid treatment cartridge |
| 23 - | Inlet connector |
| 24 - | Flow divider |
| 25 - | Actuator |
| 26 - | Control device |
| 27 - | Data processing device |
| 28 - | Memory |
| 29 - | 1st bed |
| 30 - | 1st cartridge inlet |
| 31 - | Inner fall tube |
| 32 - | 2nd bed |
| 33 - | 2nd cartridge inlet |
| 34 - | Outer fall tube |
| 35 - | Cartridge outlet |
| 36 - | Flow meter |
| 37 - | Outlet connector |
| 38 - | Sensor device |
| 39 - | Sensor interface |
| 40 - | Machine-readable token |
| 41 - | Transceiver |
| 42 - | Interface |
| 43 - | Step (determine capacity value) |
| 44 - | Current value |
| 45 - | Step (output capacity information) |
| 46 - | Step (determine and adjust settings) |
| 47 - | Step (conduct measurements) |
| 48 - | Step (determine local data) |
| 49 - | Local data |
| 50 - | Step (send local data to server) |
| 51 - | Step (identify set) |
| 52 - | Database |
| 53 - | Step (determine new consensus value) |
| 54 - | Consensus value |
| 55 - | Array |
| 56 - | Step (distribute consensus value) |
| 57 - | Step (update current value) |
| 58 - | Step (determine capacity value) |
| 59 - | Current value |
| 60 - | Step (output capacity information) |
| 61 - | Step (determine and adjust settings) |

62 - Step (conduct measurements)
63 - Step (determine local data)
64 - Local data
65 - Step (send local data to server)
66 - Step (identify set)
67 - Database
68 - Array
69 - Step (assemble new local data array)
70 - Step (distribute new local data array)
71 - Step (update current value)
72 - Step (conduct measurements)
73 - Local data
74 - Step (determine local data)
75 - Step (send local data to server)
76 - Step (identify set)
77 - Database
78 - Step (determine new consensus value)
79 - Consensus value
80 - Array
81 - Step (determine new settings)
82 - Setting
83 - Step (update capacity value)
84 - Capacity value
85 - Step (distribute setting, capacity information, consensus value)
86 - Step (adjust settings)
87 - Step (output capacity information)

**Claims**

1. Method of processing data obtained from a set of liquid treatment systems (10a-c,15a-c,20) connected in parallel to a common source (6-9) of liquid to be treated,

   wherein each liquid treatment system (10a-c,15a-c,20) comprises:

   a liquid treatment device (12a-c,17a-c,22); and
   an operational data processing system (11a-c,16a-c,21) comprising:

   at least an interface (39) to an associated sensor (38) for measuring at least one parameter for, at least in combination with setting information that is information on at least one setting (*x*;82) of the liquid treatment system (10a-c,15a-c,20), determining a value of a concentration measure (*H*) that is a measure of a concentration of at least one component changeable by the liquid treatment device (12a-c,17a-c,22) in liquid flowing through the liquid treatment device (12a-c,17a-c,22),
   wherein the operational data processing system (11a-c,16a-c,21) is configured to carry out a process (43,45,46;58,60,61;81,83,86,87) associated with operating the liquid treatment system (10a-c,15a-c,20),

   wherein the method comprises:

   obtaining from multiple ones of the liquid treatment systems (10a-c,15a-c,20) in the set local data (49;64;73), wherein the local data is selected from the group consisting of:

   (i) a local value of the concentration measure (*H*) determined by the operational data processing system (11a-c,16a-c,21) of that liquid treatment system (10a-c,15a-c,20) on the basis of at least one value of the parameter determined by the sensor (38) associated with the operational data processing system; and
   (ii) at least one value of the parameter obtained from the sensor (38) associated with the operational data processing system (11a-c,16a-c,21) of that liquid treatment system (10a-c,15a-c,20) and any setting information required to determine a value of the concentration measure (*H*) on the basis of the at least one value of the parameter;

processing the local data (49;64;73) obtained from the multiple liquid treatment systems (10a-c,15a-c,20) to determine a consensus value (54;59;79) of the concentration measure ($H$); and
providing at least one of the consensus value and a parameter value derived from the consensus value as input to the process (43,45,46;58,60,61;81,83,86,87) performed by the operational data processing system (11a-c,16a-c,21) of at least one of the liquid treatment systems (10a-c,15a-c,20) in the set.

2. Method according to claim 1,
comprising identifying the members of the set from among a larger group of liquid treatment systems (10a-c,15a-c,20).

3. Method according to claim 2,

comprising determining respective values of tagging data associated with liquid treatment systems (10a-c,15a-c,20) from which local data (49;64;73) are obtained and
identifying the members of the set based on correspondence of tagging data.

4. Method of controlling operation of a liquid treatment system (10a-c,15a-c,20) comprised in a set of liquid treatment systems (10a-c,15a-c,20) connected in parallel to a common source (6-9) of liquid to be treated,

wherein each liquid treatment system (10a-c,15a-c,20) comprises:

a liquid treatment device (12a-c,17a-c,22); and
an operational data processing system (11a-c,16a-c,21) comprising:
at least an interface (39) to an associated sensor (38) for measuring at least one parameter for, at least in combination with setting information that is information on at least one setting ($x$;82) of the liquid treatment system (10a-c,15a-c,20), determining a value of a concentration measure ($H$) that is a measure of a concentration of at least one component changeable by the liquid treatment device (12a-c,17a-c,22) in liquid flowing through the liquid treatment device (12a-c,17a-c,22),

wherein the method comprises:

the operational data processing system (11a-c,16a-c,21) of one of the liquid treatment systems (10a-c,15a-c,20) obtaining at least one value of the parameter from the sensor (38) and any setting information required to determine a value of the concentration measure ($H$) on the basis of the at least one value of the parameter,
the operational data processing system (11a-c,16a-c,21) providing local data (49;64;73) as input to a data processing system (1), wherein the local data is selected from the group consisting of:

(i) a local value of the concentration measure ($H$) determined on the basis of at least one value of the parameter obtained from the sensor (38) associated with the operational data processing system (11a-c,16a-c,21); and
(ii) the at least one value of the parameter obtained from the sensor (38) and any setting information required to determine a value of the concentration measure ($H$) on the basis of the at least one value of the parameter, and

wherein the data processing system to which the local data is provided as input is configured to obtain respective local data (49;64;73) from multiple ones of the liquid treatment systems (10a-c,15a-c,20) in the set and to use the obtained local data (49;64;73) to determine a consensus value (54;59;79) of the concentration measure ($H$);

the operational data processing system (11a-c,16a-c,21) obtaining at least one of the consensus value (54;59;79) and a parameter value derived from that consensus value (54;59;79); and
the operational data processing system (11a-c,16a-c,21) using the obtained value as input in a process (43,45,46;58,60,61;81,83,86,87) associated with operating the liquid treatment system (10a-c,15a-c,20).

5. Method according to claim 4,
wherein the step of providing local data (49;64;73) as input to a data processing system (1) comprises communicating the local data (49;64;73) to a remote server system (1) via at least one Wide Area Network (2).

6. Method according to claim 4 or 5,
wherein the step of obtaining at least one of the consensus value (54;59;79) and a parameter value derived from the consensus value (54;59;79) comprises receiving the value via at least one communication network, e.g. the at least one Wide Area Network (2).

7. Method according to claims 5 and 6,
wherein the remote server system (1) comprises the data processing system configured to obtain respective local data (49;64;73) from multiple ones of the liquid treatment systems (10a-c,15a-c,20) in the set and to use the obtained local data (49;64;73) to determine the consensus value (54;59;79) of the concentration measure (*H*).

8. Method according to any one of claims 4-7.
wherein the step of providing local data (49;64;73) as input to a data processing system (1) comprises communicating the local data (49;64;73) to the operational data processing system (11a-c,16a-c,21) of at least one of the liquid treatment systems (10a-c,15a-c,20), which operational data processing system (11a-c,16a-c,21) comprises the data processing system configured to obtain respective local data (49;64;73) from multiple ones of the liquid treatment systems (10a-c,15a-c,20) in the set and to use the obtained local data (49;64;73) to determine the consensus value (54;59;79) of the concentration measure (*H*).

9. Method according to claims 5 and 8,
wherein the remote server system (1) is configured to collate local data (49;64;73) received from the multiple liquid treatment systems (10a-c,15a-c,20) and to communicate the collated local data (49;64;73) to the operational data processing system (11a-c,16a-c,21) comprising the data processing system configured to obtain respective local data (49;64;73) from multiple ones of the liquid treatment systems (10a-c,15a-c,20) in the set and to use the obtained local data (49;64;73) to determine the consensus value of the concentration measure (*H*).

10. Method according to any one of claims 4-9,
wherein obtaining at least one of the consensus value (54;59;79) and a parameter value derived from that consensus value (54;59;79) comprises obtaining respective local data (49;64;73) from multiple ones of the liquid treatment systems (10a-c,15a-c,20) in the set and using the obtained local data (49;64;73) to determine the consensus value (54;59;79) of the concentration measure (*H*).

11. Method according to any one of the preceding claims,
wherein the process (43,45,46;58,60,61;81,83,86,87) associated with operating the liquid treatment system (10a-c,15a-c,20) comprises at least one of:

adjusting at least one of the settings (*x*;82) of the liquid treatment system (10a-c,15a-c,20); and
causing information representative of a condition of the liquid treatment system (10a-c,15a-c,20), e.g. a condition of the liquid treatment device (12a-c,17a-c,22), to be made accessible in perceptible form, e.g. via a user interface (42) at a location of the liquid treatment system (10a-c,15a-c,20).

12. System for processing data obtained from a set of liquid treatment systems (10a-c,15a-c,20),

wherein the system comprises an interface for exchanging data with multiple ones of the liquid treatment systems (10a-c,15a-c,20) in the set, and
wherein the system is configured to carry out a method according to any one of claims 1-3 or a method according to claim 11 in combination with any one of claims 1-3.

13. System for processing operational data of a liquid treatment system (10a-c,15a-c,20), comprising:

at least an interface (39) to a sensor (38) for measuring at least one parameter for, at least in combination with setting information that is information on at least one setting (*x*;82) of a liquid treatment system (10a-c,15a-c,20) comprising the sensor (38), determining a value of a concentration measure (*H*) that is a measure of a concentration of at least one component changeable by a liquid treatment device (12a-c,17a-c,22) comprised in the liquid treatment system (10a-c,15a-c,20) in liquid flowing through the liquid treatment device (12a-c,17a-c,22); and
an interface (42) for exchanging data with a further data processing system (1),
wherein the system is configured to carry out a method according to any one of claims 4-10 or a method according to claim 11 in combination with any one of claims 4-10.

14. Computer program comprising instructions which, when the program is executed by a computer comprising an interface (42) for exchanging data with multiple ones of a set of liquid treatment systems (10a-c,15a-c,20), cause the computer to carry out a method according to any one of claims 1-3 or a method according claim 11 in combination with any one of claims 1-3.

15. Computer program comprising instructions which, when the program is executed by a computer comprising at least (i) an interface (39) to a sensor (38) for measuring at least one parameter for, at least in combination with setting information that is information on at least one setting ($x$;82) of a liquid treatment system (10a-c,15a-c,20) comprising the sensor (38), determining a value of a concentration measure ($H$) that is a measure of a concentration of at least one component changeable by a liquid treatment device (12a-c,17a-c,22) comprised in the liquid treatment system (10a-c,15a-c,20) in liquid flowing through the liquid treatment device (12a-c,17a-c,22), and (ii) an interface (42) for exchanging data with a further data processing system, cause the computer to carry out a method according to any one of claims 4-10 or a method according to claim 11 in combination with any one of claims 4-10.

Fig. 1

Fig. 3

Fig. 4

Fig. 5

[Local data received]

67

64

66

Identify set

69

Assemble new local data array

68

70

Distribute new local data array

[continue]

[stop]

Fig. 6

Fig. 7

Fig. 8

Europäisches
Patentamt

European
Patent Office

Office européen
des brevets

## EUROPEAN SEARCH REPORT

**Application Number**

EP 24 20 6010

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/055750 A1 (HOEFFERLE ERICH G [US] ET AL) 20 February 2020 (2020-02-20)<br>* figures 1-6 *<br>* paragraph [0067] - paragraph [0156] *<br>----- | 1-15 | INV.<br>C02F1/00<br>C02F1/42<br>G01N33/18 |
| A | EP 2 870 472 B1 (BRITA GMBH [DE]) 26 September 2018 (2018-09-26)<br>* figures 1-5 *<br>* paragraph [0075] - paragraph [0123] *<br>----- | 1-15 | ADD.<br>C02F1/28 |

**TECHNICAL FIELDS SEARCHED (IPC)**

C02F
G01N

The present search report has been drawn up for all claims

| Place of search | Date of completion of the search | Examiner |
|---|---|---|
| The Hague | 29 January 2025 | Châtellier, Xavier |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P4C01)

## ANNEX TO THE EUROPEAN SEARCH REPORT
## ON EUROPEAN PATENT APPLICATION NO.

EP 24 20 6010

This annex lists the patent family members relating to the patent documents cited in the above-mentioned European search report.
The members are as contained in the European Patent Office EDP file on
The European Patent Office is in no way liable for these particulars which are merely given for the purpose of information.

29-01-2025

| Patent document cited in search report | | Publication date | Patent family member(s) | | Publication date |
|---|---|---|---|---|---|
| US 2020055750 | A1 | 20-02-2020 | AU | 2019317516 A1 | 28-01-2021 |
| | | | CA | 3106069 A1 | 13-02-2020 |
| | | | EP | 3833636 A1 | 16-06-2021 |
| | | | IL | 280251 A | 01-03-2021 |
| | | | SG | 11202100289Y A | 25-02-2021 |
| | | | US | 10501343 B1 | 10-12-2019 |
| | | | US | 2020055750 A1 | 20-02-2020 |
| | | | US | 2020216333 A1 | 09-07-2020 |
| | | | US | 2023025170 A1 | 26-01-2023 |
| | | | WO | 2020033011 A1 | 13-02-2020 |
| EP 2870472 | B1 | 26-09-2018 | AU | 2013285426 A1 | 05-02-2015 |
| | | | CN | 104603614 A | 06-05-2015 |
| | | | DK | 2870472 T3 | 12-11-2018 |
| | | | EP | 2870472 A1 | 13-05-2015 |
| | | | ES | 2696981 T3 | 21-01-2019 |
| | | | PL | 2870472 T3 | 28-02-2019 |
| | | | RU | 2015103676 A | 27-08-2016 |
| | | | TW | 201418708 A | 16-05-2014 |
| | | | WO | 2014006128 A1 | 09-01-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013166069 A1
- WO 2009101188 A1
- WO 2016012537 A1
- WO 2014006128 A1